(19) Europäisches Patentamt
European Patent Office
Office européen des brevets

(11) Publication number: **0 305 184 B1**

# (12) EUROPEAN PATENT SPECIFICATION

(45) Date of publication of patent specification: **27.04.94**

(51) Int. Cl.5: **C07D 487/04**, C07D 239/48, C07D 401/04, C07D 239/47, A61K 31/505, //(C07D487/04, 239:00,209:00)

(21) Application number: **88307893.3**

(22) Date of filing: **25.08.88**

## (54) Pyrimidines or their pharmaceutically acceptable salts thereof.

(30) Priority: **26.08.87 JP 210170/87**

(43) Date of publication of application:
**01.03.89 Bulletin 89/09**

(45) Publication of the grant of the patent:
**27.04.94 Bulletin 94/17**

(84) Designated Contracting States:
**AT BE CH DE FR GB IT LI LU NL SE**

(56) References cited:
**EP-A- 0 188 094**
**EP-A- 0 257 102**
**CH-A- 358 426**
**US-A- 4 215 216**

**CHEMICAL ABSTRACTS, vol. 83, 1975, page 87, no. 22902m, Columbus, Ohio, US; V. SICHO et al.: "Effect of antivitamins on the microbiological biosynthesis of vitamins. V. Stimulation of thiamine biosynthesis in the microorganism Saccharomyces cerevisiae"**

(73) Proprietor: **MITSUI PHARMACEUTICALS, INC.**
**12-2, Nihonbashi 3-chome**
**Chuo-ku**
**Tokyo 103(JP)**

Proprietor: **MITSUI PETROCHEMICAL INDUSTRIES, LTD.**
**2-5, Kasumigaseki 3-chome**
**Chiyoda-ku**
**Tokyo 100(JP)**

(72) Inventor: **Awaya, Akira**
**1541, Yabe-cho, Totsuka-ku**
**Yokohama-shi Kanagawa-ken(JP)**
Inventor: **Horikomi, Kazutoshi**
**103, Hagiwara-cho 1-chome**
**Mobara-shi Chiba-ken(JP)**
Inventor: **Sasaki, Tadayuki**
**2142, Tougou**
**Mobara-shi Chiba-ken(JP)**
Inventor: **Kobayashi, Hisashi**
**2141, Tougou**
**Mobara-shi Chiba-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

JOURNAL OF THE CHEMICAL SOCIETY (C), 1967, pages 1172-1178, London, GB; R.S. SHADBOLT et al.: "Pyrimidines. Part II.1 Nucleophilic substitution reactions of ethyl 4-chloro-2-methylthiopyrimidine-5-carboxylate"

CHEMICAL ABSTRACTS, vol. 90, 1979, page 640, no. 121529w, Columbus, Ohio, US; V.G. GRANIK et al.: "Acetals of lactams and acid amides. XXVII. New synthesis of pyrido[2,3-d]pyrimidine derivatives"

CHEMICAL ABSTRACTS, vol. 86, 1977, page 552, no. 121363e, Columbus, Ohio, US; & JP-A-76 100 088

CHEMICAL ABSTRACTS, vol. 109, 1988, page 617, no. 110363x, Columbus, Ohio, US; K. HASHIZUME et al.: "Synthesis of 2-butylthiothiamine and its inhibitory effect on the growth of Saccharomyces carlsbergenis"

Inventor: **Mizuchi, Akira**
**11-6, Toubudai 3-chome**
**Mobara-shi Chiba-ken(JP)**
Inventor: **Nakano, Takuo**
**2231-20, Kamigo-cho**
**Totsuka-ku**
**Yokohama-shi Kanagawa-ken(JP)**
Inventor: **Tomino, Ikuo**
**2-7, Misono 1-chome**
**Ohtake-shi Hiroshima-ken(JP)**
Inventor: **Araki, Shintaro**
**4-9, Waki 2-chome, Waki-cho**
**Kuga-gun Yamaguchi-ken(JP)**
Inventor: **Takesu, Mitsuyuki**
**12-2, Shozoku-cho 5-chome**
**Iwakuni-shi Yamaguchi-ken(JP)**
Inventor: **Kato, Koji**
**5-3, Waki 3-chome, Waki-cho**
**Kuga-gun Yamaguchi-ken(JP)**
Inventor: **Yokoyama, Keiichi**
**103-9, Misho 2-chome**
**Iwakuni-shi Yamaguchi-ken(JP)**

(74) Representative: **Myerscough, Philip Boyd et al**
**J.A.KEMP & CO.**
**14, South Square**
**Gray's Inn**
**London WC1R 5LX (GB)**

## Description

This invention relates to novel pyrimidines and pharmaceutically acceptable salts thereof, to a process for their preparation and to therapeutic agents and pharmaceutical compositions comprising them.

JP-B-23,394/1971 discloses aminopyrimidines represented by the formula

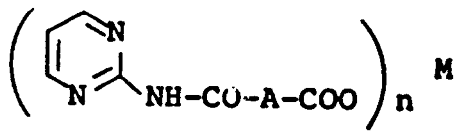

wherein A represents an alkylene group having up to 16 carbon atoms, or a lower alkylene group substituted by an amino group or a $C_{2-5}$ acylamino group, M represents H, Na, K, $NH_4$, Mg, Ca or an organic basic ammonium salt, and n is equal to the atomic valency of M,
which have interesting therapeutic activity, particularly as anti-melanchoric and psychoanaleptic agents in the field of psychosis.

JP-B-22044/1976 discloses dichloro-lower aliphatic carboxylic acid salts of 2-isopropylaminopyrimide, such as 2-isopropylaminopyrimidine dichloroacetate, which are useful as therapeutic agents for neurological diseases.

JP-A-100477/1977 (JP-B-28548/1984) discloses 2-isopropylaminopyrimidine phosphate which is useful as a therapeutic agent for a neurological disease.

JP-B-157575/1979 discloses a process for producing 2-chloropyrimidine in a high yield. A working example in this patent publication describes the preparation of 2-chloropyrimidine with a yield of 69 %.

JP-A-393/1980 discloses a process for producing 2-isopropylaminopyrimidine in a high yield. A working example of this patent publication describes the preparation of 2-isopropylaminopyrimidine with a yield of 60 %.

JP-A-122768/1980 discloses that a hydroxy derivative of 2-isopropylaminopyrimidine represented by the formula

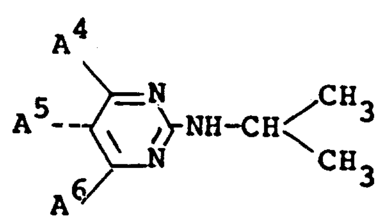

wherein $A^4$, $A^5$ and $A^6$ each represent H or OH, and at least one of them represents OH,
is useful in the field of nerve regeneration and for the treatment of myodystrophy.

JP-A-145670/1980 discloses that 2-isopropylaminohalogenopyrimidines represented by the formula

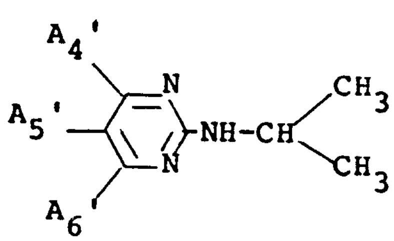

wherein $A_4'$, $A_5'$ and $A_5'$ each represent H or a halogen atom, and at least one of them is a halogen atom,
are useful for the treatment of various neurological diseases and myodystrophy.

JP-A-145,671/1980 discloses a process for producing a hydroxy derivative of 2-isopropylaminopyrimidine.

JP-A-151,571/1980 discloses that 2-isopropylamino-5-halogenopyrimidines are interesting in the treatment of neurological diseases.

JP-A-10177/1981 discloses a process for producing 2-isopropylaminopyrimidine substantially in a quantitative yield by aminolyzing 2-methylsulfonylpyrimidine with isopropylamine.

JP-A-26880/1981 discloses a process for producing 2-isopropylaminopyrimidine which comprises reacting bis(isopropylguanidine) sulfate with 1,1,3,3-tetraethoxypropane.

JP-A-90,013/1981 describes a therapeutic agent for myodystropy, myopathy, muscle rigidity and/or dysfunction of neuro-muscular transmission comprising a substituted derivative of pyrimidine or a therapeutically acceptable salt or metabolite thereof as active ingredient. However, it merely discloses various salts such as the orthophosphate of 2-isopropylaminopyrimidine as active compound.

JP-A-65873/1986 discloses that 2-piperazinopyrimidine derivatives of the formula

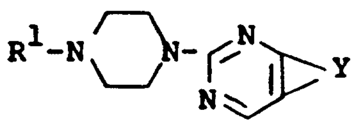

wherein $R^1$ is H or aralkyl, and Y is a divalent organic group defined in the patent publication, are useful as herbicides for paddies and upland farms.

The present inventors previously provided a novel therapeutic agent for neurological diseases comprising a specified 2-piperazinopyrimidine derivative or pharmaceutically acceptable salt thereof (WO87/04928).

The present invention seeks to provide novel pyrimidines and pharmaceutically acceptable salts thereof and therapeutic agents which comprise the above novel compounds for neurological or cerebral diseases which: have the effect of regenerating and repairing nerve cells; can be applied to disorders of peripheral nerves; can be applied to diseases of central nerves which are different from psycosis and in which abnormality in the operating system of the metabolic system of chemical transmitters is regarded as being primarily involved; have the effect of improving and restoring learning and memory; and

which comprises a comprehensively excellent and useful compound having pharmacological activity suitable for the treatment of neurological diseases or cerebral diseases with little side effects such as liver trouble.

Accordingly the present invention provides a compound which is a pyrimidine derivative of formula (I)

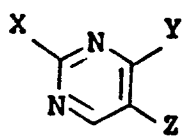

... (I)

wherein X represents either a group of formula (I)-1

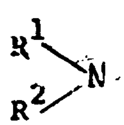

... (I)-1

wherein $R^1$ represents a hydrogen atom or an alkyl group having 1 to 4 carbon atoms, $R^2$ represents a piperidyl group which may be substituted by a $C_{1-4}$ alkyl group or a phenethyl, cyclohexyl, phenyl or benzyl group or an alkyl group having 1 to 4 carbon atoms which may be substituted by a piperidino group, or $R^1$ and $R^2$ together with the nitrogen atom to which they are bonded may form a heterocyclic ring selected from

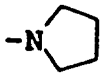, 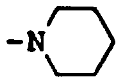, 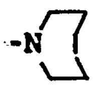, 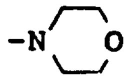, 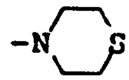, 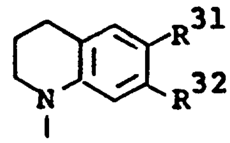,

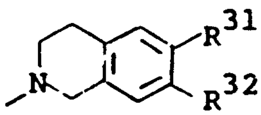 and 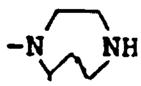

together

wherein $R^{31}$ and $R^{32}$ are identical or different and each represents a hydrogen atom or an alkoxy group having 1 to 4 carbon atoms, and the heterocyclic group may optionally be substituted by a phenyl, benzyl, phenylthio, cyano or alkoxy carbonyl group having 1 to 4 carbon atoms or mono-substituted by the group or mono- to penta-substituted by a $C_{1-4}$ alkyl group, or substituted by a $C_{3-5}$ polymethylene group on the adjoining ring-member carbons, or (ii) a group of formula (I)-2

$-S-R^4$ (I)-2

wherein $R^4$ represents an alkyl group having 1 to 4 carbon atoms, Y represents an amino group or a substituted amino group mono- or disubstituted by a $C_{1-4}$ alkyl group, and Z represents a methyl group substituted by a $C_{2-5}$ alkoxycarbonyl group or an alkorycarbonyl group having 2 to 5 carbon atoms, or Y and Z together may form a divalent group -Y-Z- of formula

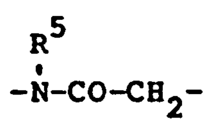

wherein $R^5$ represents an alkyl group having 1 to 4 carbon atoms which may be substituted by an alkoxy group having 1 to 4 carbon atoms, or a group of the following formula

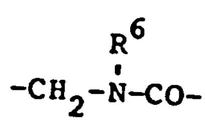

wherein $R^6$ represents an alkyl group having 1 to 4 carbon atoms provided that when Y is an amino group or a substituted amino group mono- or di-substituted by a $C_{1-4}$ alkyl group, X is

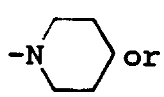 or 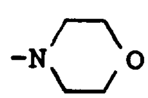;

or their pharmaceutically acceptable salts.

A $C_{1-4}$ alkyl group may be linear or branched, for example methyl, ethyl, n-propyl, isopropyl, n-butyl, sec-butyl, isobutyl and t-butyl.

A $C_{1-4}$ alkoxycarbonyl group is for example a methoxycarbonyl, ethoxycarbonyl, n-propoxycarbonyl, isopropoxycarbonyl, n-butoxycarbonyl, isobutoxycarbonyl, secbutoxycarbonyl or tert-butoxycarbonyl group.

A polymethylene group having 3 to 5 carbon atoms is for example, a trimethylene, tetramethylene or pentamethylene group. These groups may form 5-membered, 6-membered and 7-membered rings respectively together with the adjacent ring-member carbons to which they are bonded.

Examples of the group represented by formula (I)-2 are methylthio, ethylthio, n-propylthio, isopropylthio, n-butylthio, sec-butylthio and t-butylthio groups.

A $C_{2-5}$ alkoxycarbonyl group is for example, a methoxycarbonyl, ethoxycarbonyl, n-propoxycarbonyl, isopropoxycarbonyl, n-butoxycarbonyl or sec-butoxycarbonyl group.

Examples of compounds of the invention are shown below.

(A) Compounds of formula (I) in which -Y-Z- represents

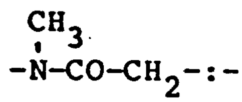

(100)

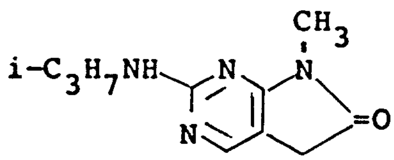

(102) Hydrochloride of (100)
(104) Maleate of (100)

(106)

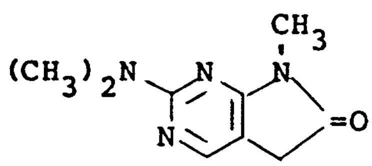

(108) Maleate of (106)

(110)

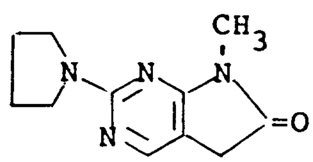

(112) Maleate of (110)

(114)

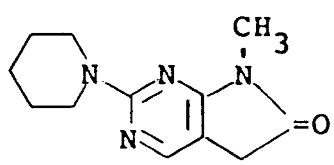

(116) Hydrochloride of (114)
(118) Maleate of (114)

(500)

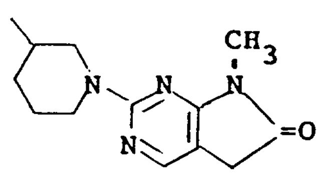

(502) Hydrochloride of (500)

(120) 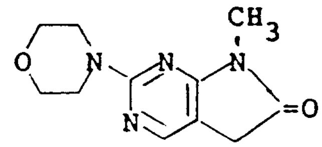

(122) Hydrochloride of (120)

(124) 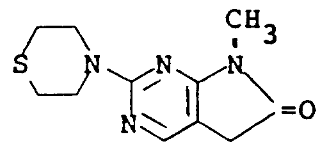

(126) Hydrochloride of (124)

(128) 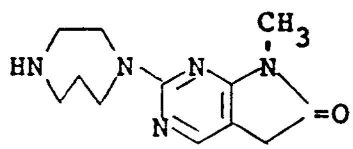

(129) Hydrochloride of (128)

(134) 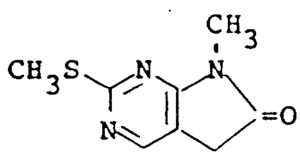

(144) 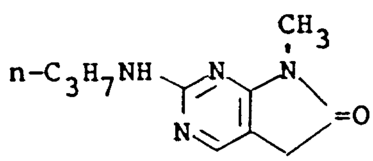

(146) 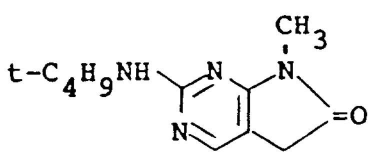

(147) 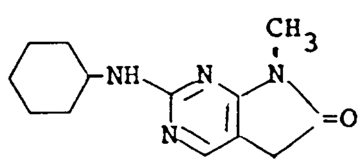

(148) 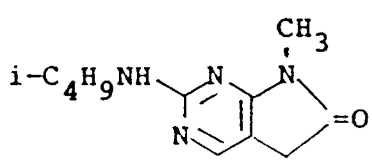

(152)

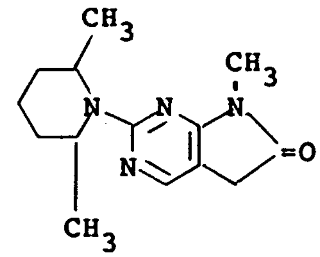

(154)

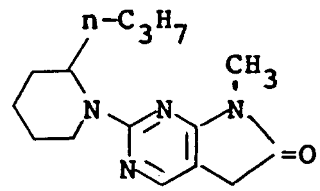

(156)

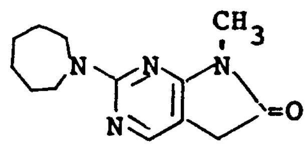

(158)

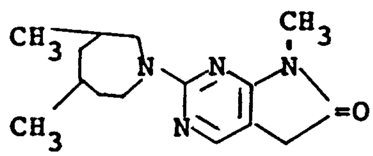

(160)

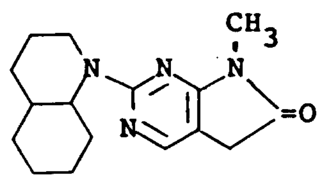

(162)

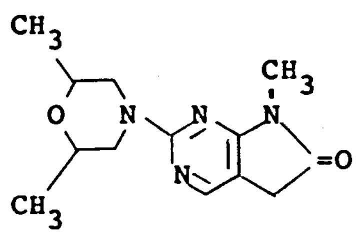

(164)

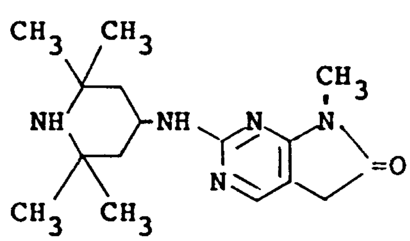

(166) 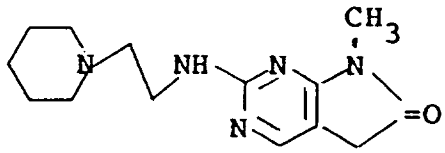

(174) 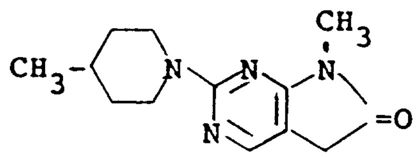

(175) Hydrochloride of (174)

(176) 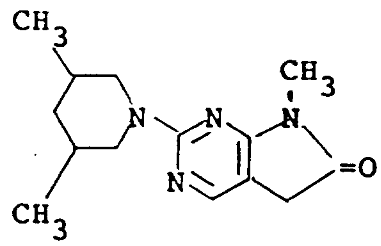

(177) Hydrochloride of (176)

(178) 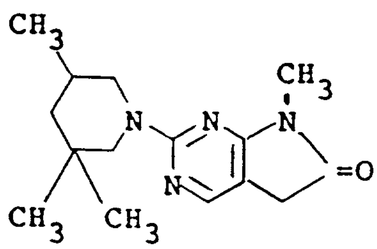

(544) 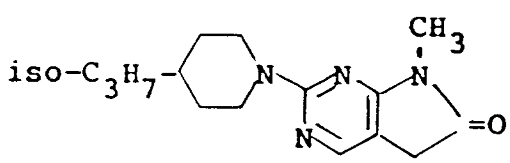

(546) Hydrochloride of (544)

(548) 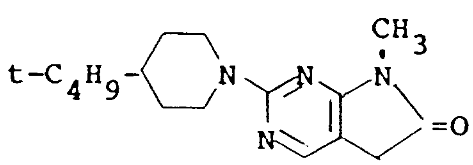

(550) Hydrochloride of (548)

(552)

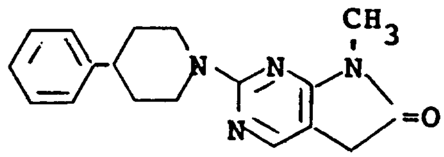

(554) Hydrochloride of (552)

(556)

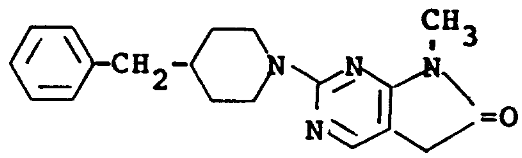

(557) Hydrochloride of (556)

(558)

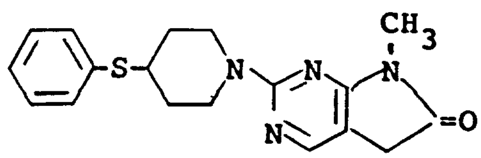

(559) Hydrochloride of (558)

(560)

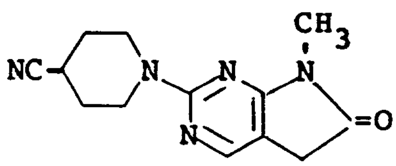

(562) Hydrochloride of (560)

(564)

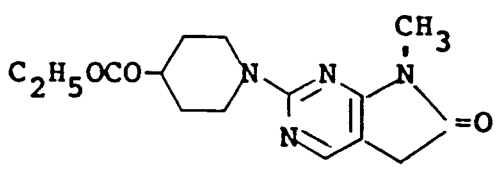

(566) Hydrochloride of (564)

(568)

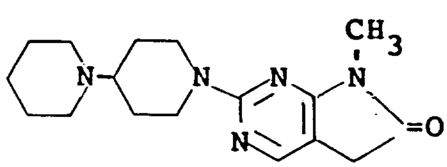

(570) Hydrochloride of (568)

(572) 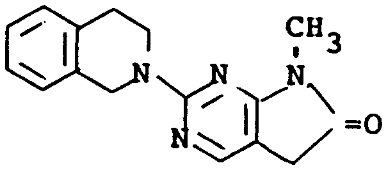

(574) Hydrochloride of (572)

(575) 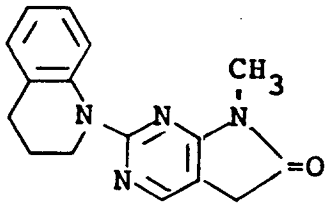

(576) 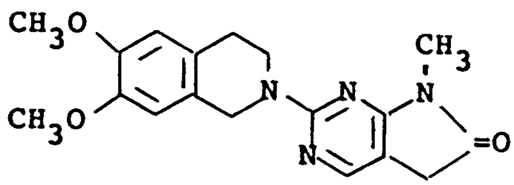

(578) Hydrochloride of (576)

(580) 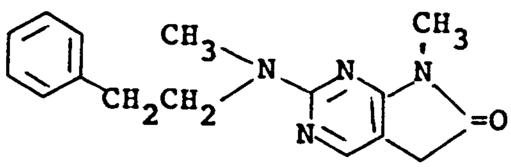

(582) Hydrochloride of (580)

(584) 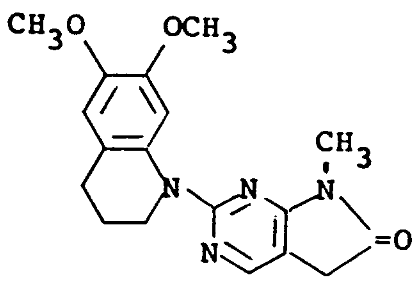

(180) 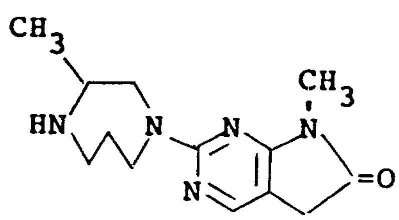

(182) 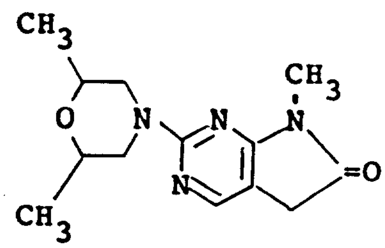

(B) Compounds of formula (I) in which -Y-Z- represents

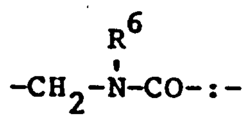

(200) 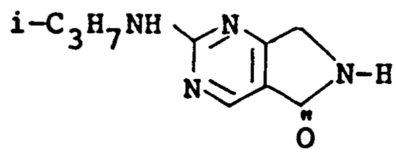

(202) 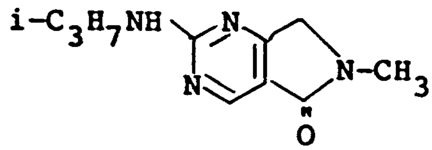

(204) Hydrochloride of (202)

(206) 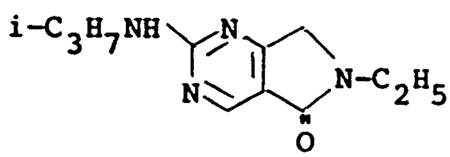

(208) 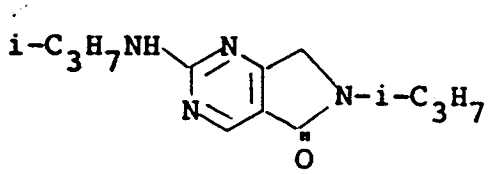

(210) 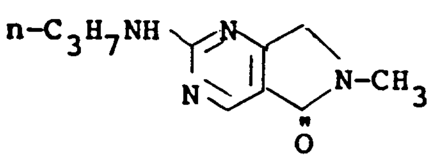

(211) Hydrochloride of (210)

(212) 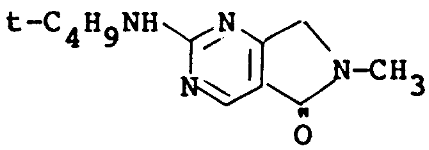

(214) 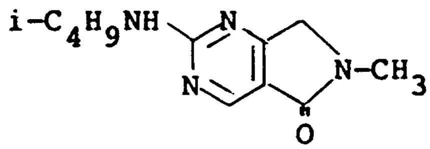

(215) Hydrochloride of (214)

(216) 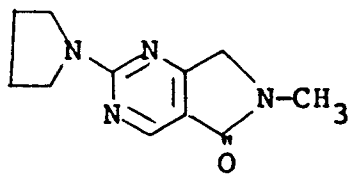

(218) 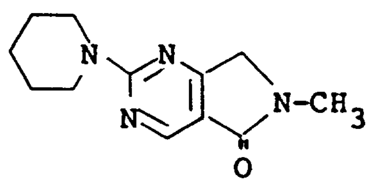

(584) 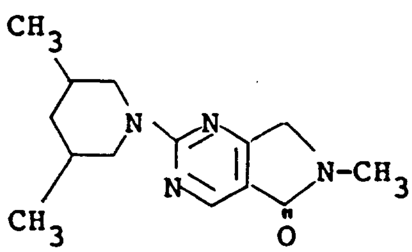

(220) 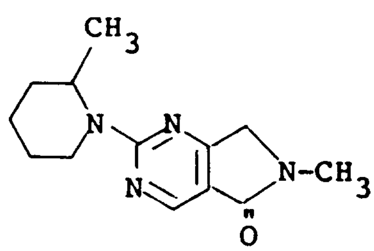

(222) 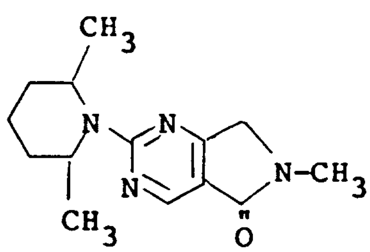

(224) 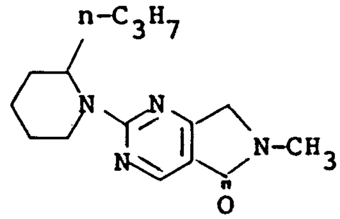

(226) 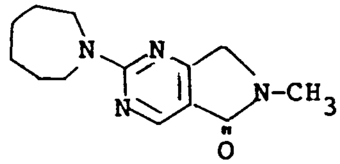

(228) 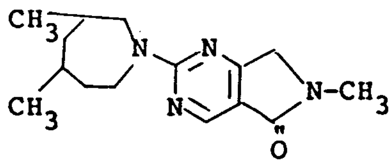

(230) 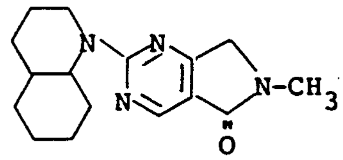

(232) 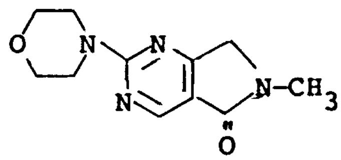

(234) 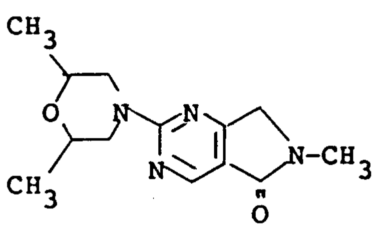

(236) 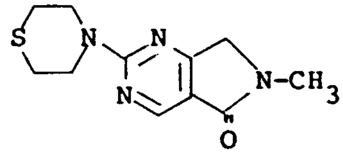

(238) 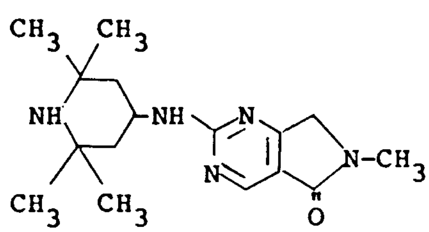

(239) Hydrochloride of (238)

(240) 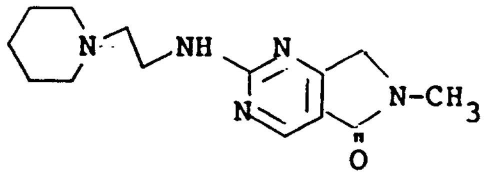

(241) Hydrochloride of (240)

(C) Compounds of formula (I) in which Y and Z, independently from each other, represent a monovalent group:-

(400) 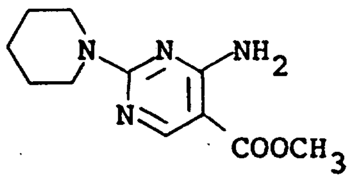

(402) Hydrochloride of (400)

(404) 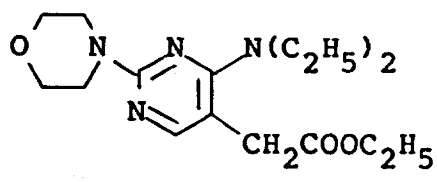

(406) Maleate of (404)

The compounds provided by this invention can be produced by known processes, particularly the processes described in JP-A-140568/1986 and 87627/1986, or by treating the intermediates obtained by these processes, by a known method (for example, by reductive elimination of the protective group). Examples 1 to 14 given hereinbelow describe the processes for producing the compounds of formula (I) in detail.

For example, the compounds (I) of this invention can be produced more specifically by the following proceses.

(a) To produce compounds of the following formula (I)-A

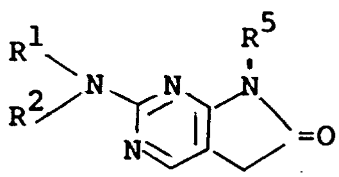 ... (I)-A

wherein $R^1$, $R^2$ and $R^5$ are as defined in formula (I),

a compound of the following formula (V)

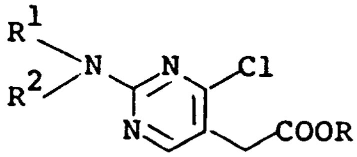 ... (V)

wherein $R^1$ and $R^2$ are as defined with regard to formula (I) above, and R is an alkyl group having 1 to 4 carbon atoms,

is reacted with an amine of the following formula (VI)

$R^5NH_2$ (VI)

wherein $R^5$ is as defined with regard to formula (I).

This reaction beginning with the starting material can be carried out in accordance with the following Reaction Scheme 1.

## Reaction Scheme 1

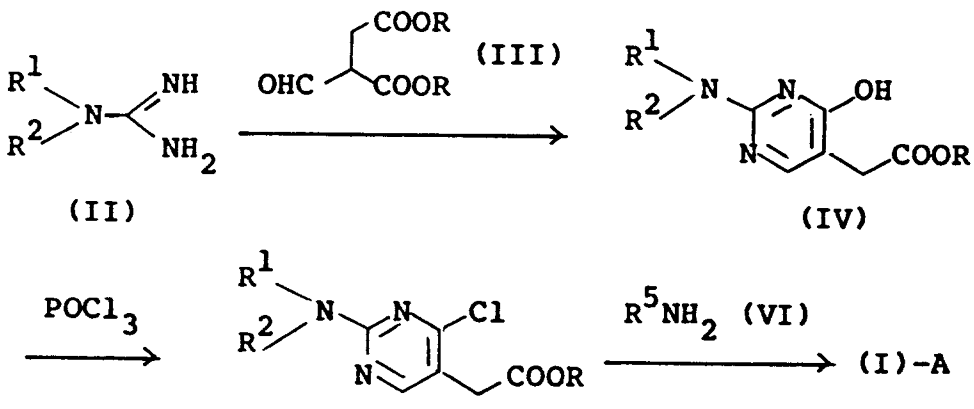

(II) (IV) (V) (I)-A

This process can be carried out, for example, as follows. Compounds (II) and (III) are reacted at a temperature of 0 to 100 °C for 0.5 to 10 hours in a reaction medium such as water, methanol, ethanol, tetrahydrofuran or dimethylformamide to form compound (IV). Compound (IV) is reacted with phosphorus oxychloride without a solvent or in an inert solvent such as dichloroethane to form compound (V). Then, compound (V) is reacted with compound (VI) at a temperature of 80 to 150 °C in an alcohol solvent such as isopropanol and ethanol to produce compound (I)-A.

The intermediate (IV) used in Reaction Scheme 1 can also be produced in accordance with Reaction Scheme 1'.

## Reaction Scheme 1'

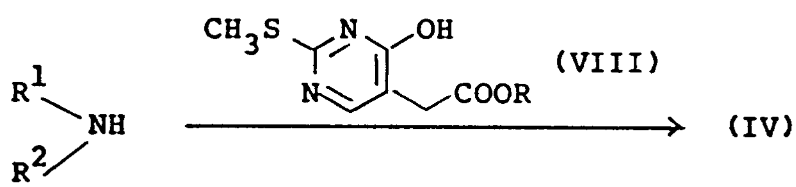

(VII) (IV)

The process in Reaction Scheme 1' may be carried out by reacting compounds (VII) and (VIII) at a temperature of 100 to 200 °C in an alcohol solvent such as butanol and amyl alcohol to produce compound (IV). Compound (VIII) can also be produced by Reaction Scheme 1 except that S-methylisothiourea is used instead of compound (II).

Compounds encompassed within formula (I)-A are compounds Nos. 100, 106, 110, 114, 500, 120, 124, 128, 144, 146, 148, 150, 152, 154, 156, 158, 160, 162, 164, 166, 174, 176, 178, 544, 548, 552, 556, 558, 560, 564, 568, 572, 575, 576, 580, 582, 180 and 182.

(b) To produce compounds of formula (I)-B

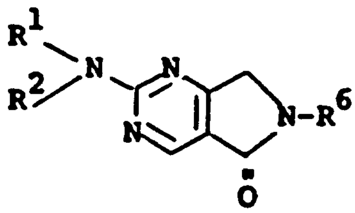

... (I)-B

wherein $R^1$, $R^2$ and $R^6$ are as defined with regard to formula (I),

($b^1$) a compound of formula (XII)

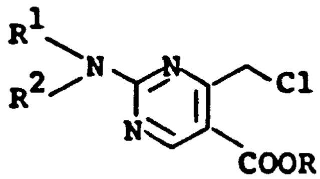

... (XII)

wherein $R^1$ and $R^2$ are as defined with regard to formula (I), and R represents an alkyl group having 1 to 4 carbon atoms,

is reacted with a compound of the following formula (XIII)

$R^6NH_2$ (XIII)

wherein $R^6$ is as defined with regard to formula (I), or

($b^2$) a compound of the following formula (XIV)

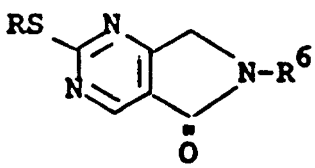

... (XIV)

wherein $R^6$ is defined with regard to formula (I), and R is an alkyl group having 1 to 4 carbon atoms,

is reacted with a compound of the following formula (XV)

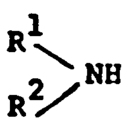

... (XV)

wherein $R^1$ and $R^2$ are as defined with regard to formula (I).

The above reaction may be carried out in accordance with Reaction Scheme 2 or 3 beginning with the starting material.

## Reaction Scheme 2

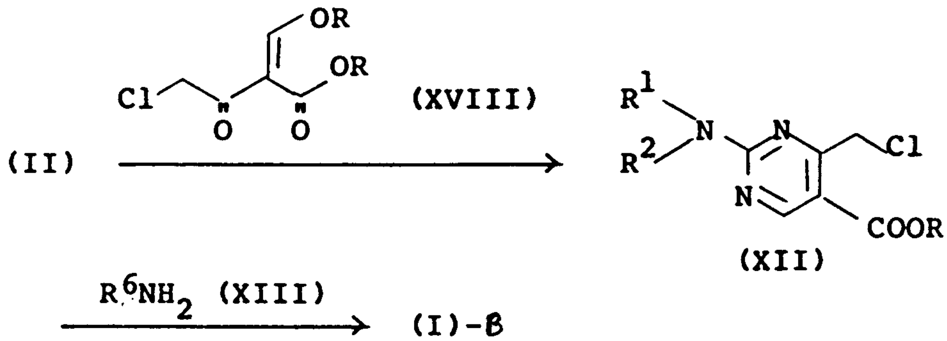


(II) (XII) (I)-B

## Reaction Scheme 3

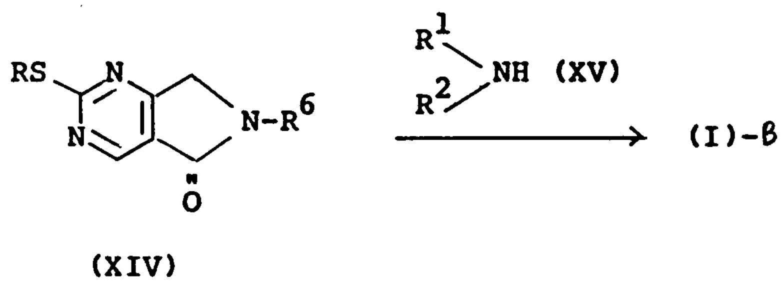


(XIV) (I)-B

The process of Reaction Scheme 2 can be carried out, for example, as follows:

Compounds (II) and (XVIII) (in which R is an alkyl group having 1 to 4 carbon atoms) are reacted at a temperature of 0 to 100 °C, preferably for 0.5 to 10 hours, in a reaction solvent such as water, methanol, ethanol, tetrahydrofuran or dimethylformamide to form compound (XII). Compound (XII) is reacted with compound (XIII) at a temperature of 0 to 150 °C, preferably 0.5 to 20 hours, in a solvent such as water, an alcohol (e.g., methanol or ethanol), tetrahydrofuran, dimethylformamide, toluene or xylene to produce compound (I)-B.

The process of Reaction Scheme 3 may be carried out, for example, as follows:

Compounds (XIV) and (XV) are reacted at a temperature of 80 to 150 °C in an alcohol solvent such as butanol or amyl alcohol to form compound (I)-B. Compound (XIV) can be produced in the same way as in Reaction Scheme 3 except that S-methylisothiourea is used instead of compound (II).

Compounds encompassed within formula (I)-B are compounds Nos. 200, 202, 206, 208, 210, 212, 214, 216, 218, 584, 220, 222, 224, 226, 228, 230, 232, 234, 236, 238, and 240.

(c) To produce compounds of the following formula (I)-C

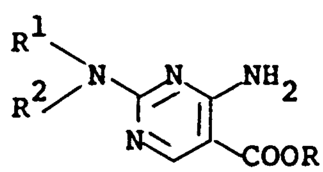

... (I)-C

wherein $R^1$ and $R^2$ are as defined with regard to formula (I) and R represents an alkyl group having 1 to 4 carbon atoms,

a compound represented by the following formula (XVI)

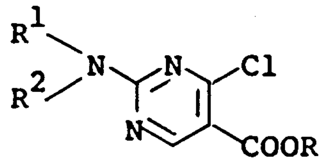

... (XVI)

wherein $R^1$, $R^2$ and R are as defined above, is reacted with ammonia.

The reaction can be shown by the following Reaction Scheme 4.

**Reaction Scheme 4**

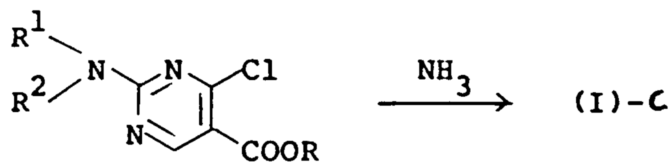


(XVI)

Reaction Scheme 4 may be carried out as follows:-

Compound (XVI) can be produced by the same procedure as in the preparation of compound (V) in accordance with Reaction Scheme 1 except that a dialkyl 2-ethoxymethylenemalonate such as diethyl 2-ethoxymethylenemalonate is used instead of compound (III). The reaction of compounds (XVI) and $NH_3$ can also be carried out as in Reaction Scheme 1 to produce compound (I)-C.

An example of the compound of formula (I)-C is compound No. 400.

(d) To produce compounds of the following formula (I)-D

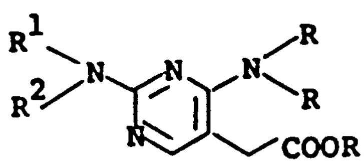

... (I)-D

wherein $R^1$ and $R^2$ are as defined with regard to formula (I), and R's, independently from each other, represent an alkyl group having 1 to 4 carbon atoms,
a compound of formula (V) wherein $R^1$ and $R^2$ together with the nitrogen atom to which they are attached form a

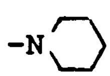 or a 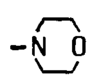

group is reacted with a compound of the following formula (XVII)

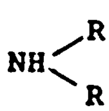

..... (XVII)

wherein R's, independently from each other, represent an alkyl group having 1 to 4 carbon atoms.

The above reaction may be shown by the following Reaction Scheme 5.

**Reaction Scheme 5**

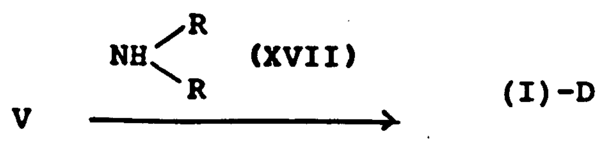


Compound (I)-D may be produced in accordance with Reaction Scheme 5 by reacting compound (VI) with compound (XVII) instead of compound (VI) in accordance with Reaction Scheme 1.

An example of the compound of formula (I)-D is compound No. 404.

The pharmaceutically acceptable salt of the compound of formula (I) may be produced in accordance with the following procedure. The hydrochloride may be produced by dissolving the corresponding compound of formula (I) in a solvent such as toluene, ether, ethanol or ethyl acetate, and blowing hydrogen chloride gas into the solution or adding concentrated hydrochloric acid to the solution. Examples of the hydrochloride are compounds Nos. 102, 114, 502, 122, 126, 129, 175, 177, 546, 550, 554, 557, 562, 566, 570, 574, 578, 582, 204, 211, 215, 239, 241 and 400.

Corresponding maleates and p-toluenesulfonates can be obtained in the same way by using maleic acid and p-toluenesulfonic acid instead of hydrochloride acid. Examples of such salts are maleates Nos. 104, 108, 112, 118, 138 and 404.

In accordance with this invention, the compounds of formula (I) and the pharmaceutically salts thereof have been found to be useful as active ingredient in therapeutic agents for use in the treatment of neurological diseases.

The compounds of formula (1) and the pharmaceutically acceptable salts thereof are used normally in the form of a pharmaceutical composition, and administered through various routes, for example oral, subcutaneous, intramuscular, intravenous, intrarhinal and intrarectal routes and also by transmission through the skin.

The present invention also provides a pharmaceutical composition comprising a pharmaceutically acceptable diluent in association with a compound of formula (I) or a pharmaceutically acceptable salt thereof as active ingredient. The pharmaceutically acceptable salt includes, for example, acid addition salts and quaternary ammonium (or amine) salts.

Examples of the pharmaceutically acceptable salts of the compounds of formula (1) include salts formed from acids capable of forming pharmaceutically acceptable non-toxic acid-addition salts containing anions, such as hydrochlorides, hydrobromides, sulfates, bisulfites, phosphates, acid phosphates, acetates, maleates, fumarates, succinates, lactates, tartrates, benzoates, citrates, gluconates, glucanates, methanesulfonates, p-toluenesulfonates and naphthalenesulfonates or their hydrates, and quaternary ammonium (or amine) salts or their hydrates.

The composition of this invention may be formulated into tablets, capsules, powders, granules, troches, cachet wafer capsules, elixirs, emulsions, solutions, syrups, suspensions, aerosols, ointments, aseptic injectables, molded cataplasmas, tapes, soft and hard gelatin capsules, suppositories, and aseptic packed powders. Examples of the pharmaceutically acceptable carrier include lactose, glucose, sucrose, sorbitol, mannitol, corn starch, crystalline cellulose, gum arabic, calcium phosphate, alginates, calcium silicate, microcrystalline cellulose, polyvinyl pyrrolidone, tragacanth gum, gelatin, syrup, methyl cellulose, carboxymethyl cellulose, methylhydroxybenzoic acid esters, propylhydroxybenzoic acid esters, talc, magnesium stearates, inert polymers, water and mineral oils.

Both solid and liquid compositions may contain the aforesaid fillers, binders, lubricants, wetting agents, disintegrants, emulsifying agents, suspending agents, preservatives, sweetening agents and flavoring agents. The composition of this invention may be formulated such that after administration to a patient, the active compound is released rapidly, continuously or slowly.

In the case of oral administration, the active ingredient is mixed with a carrier or diluent and formed into tablets or capsules, for example. In the case of parenteral administration, the active ingredient is dissolved in a 10 % aqueous solution of glucose, isotonic salt water, sterilized liquid, for example sterilized water, and enclosed in vials or ampoules for intravenous instillation or injection or intramuscular injection. Advantageously, a dissolution aid, a local anesthetic agent, a preservative and a buffer may also be included into the medium. To increase stability, it is possible to lyophilize the present composition after introduction into a vial or ampoule. Another example of parenteral administration is the administration of the pharmaceutical

composition through the skin as an ointment or a cataplasm. In this case, a molded cataplasm or a tape is advantageous.

The composition of this invention contains 0.1 to 2000 mg, more generally 0.5 to 1000 mg, of the active component for each unit dosage form.

The active ingredient is effective over a wide dosage range. For example, the amount of active ingredient administered for one day is usually from 0.003 mg/kg to 100 mg/kg. The amount of active ingredient to be actually administered is determined by a physician depending, for example, upon the type of the compound administered, and the age, body weight and reaction condition of the patient and the administration route.

The above dosage range, therefore, does not limit the scope of the invention. The suitable number of administrations is 1 to 6, usually 1 to 4, daily.

The activce ingredient by itself is an effective therapeutic agent for disorders of the peripheral nervous system and the central nervous system. If required, it may be administered in combination with at least one other equally effective drug. Examples of such an additional drug are gangliosides, mecobalamin and isaxonine.

EXAMPLE 1

2-iso-Propylamino-5,6-dihydro-7-methyl-6-oxo-(7H)pyrrolo[2,3-d]pyrimidine (compound No. 100):-

Phosphorus oxychloride (26.2 g) was added to 2.26 g (9.45 mmoles) of ethyl 2-isopropylamino-4-hydroxypyrimidine-5-acetate, and the mixture was heated under reflux for 3 hours. The reaction mixture was concentrated under reduced pressure, and chloroform and ice water were added. It was then neutralized with sodium hydrogen carbonate. The chloroform layer was separated, and the solvent was evaporated. The residue was purified by silica gel column chromatography to give 1.80 g (yield 74 %; melting point 67-71 °C) of ethyl 2-isopropylamino-4-chloropyrimidine-5-acetate. To the product were added 1.05 g (13.5 mmoles) of a 40 % methanol solution of methylamine and 10 ml of ethanol were added, and the mixture was reacted at 120 °C for 7 hours in an autoclave. Water was added, and the mixture was extracted with chloroform. The solvent was evaporated. The residue was purified by silica gel column chromatography to give 0.50 g (yield 35 %) of the desired compound.

Melting point: 120-123 °C.

$^1$H-NMR spectrum ($CDCl_3$ solution, δppm): 1.28(6H, d, J=5Hz), 3.20(3H, s), 3.44 (2H, s), 4.20(1H, quint. J=5Hz), 5.0 (1H, br.), 7.90(1H, s).

In the same way as above, the following compounds were produced.

| Compound No. | Yield (%) | Melting point (°C) | $^1$H-NMR spectrum ($CDCl_3$ solution, δ ppm) |
|---|---|---|---|
| 106 | 56 | 150-153 | 3.20(9H, s), 3.40(2H, s), 7.92(1H, s). |
| 110 | 71 | 190-192 | 1.9-2.1(4H, m), 3.20(3H, s), 3.40(2H, s), 3.5-3.7(4H, m), 7.92(1H, s). |
| 114 | 65 | 120-122 | 1.68(6H, m), 3.22(3H, s), 3.44(2H, s), 3.80(4H, m), 7.92 (1H, s). |
| 120 | 43 | 172.0-173.5 | 3.20(3H, s), 3.44(2H, s), 3.80(8H, s), 7.93(1H, s). |
| 124 | 37 | 181-183 (decomp.) | 2.67(4H, m), 3.20(3H, s), 3.44(2H, s), 4.16(4H, m), 7.92 (1H, s). |
| 128* | 81 | 167-167 (decomp.) | 2.1-2.3(2H, m), 3.16(3H, s), 3.1-3.4(4H, m), 3.47(2H, s), 3.8-4.2(4H, m), 7.94(1H, s). |
| 500 | 55 | 95-96 | 0.96(3H, d, J=7.0Hz), 1.0-2.0(5H, m), 2.56(1H, d.d, J=10.8Hz), 2.84(1H, m), 3.21(3H, s), 3.41(2H, s), 4.61 (2H, br.d, J=12.6Hz), 9.90(1H, s). |

- to be continued -

| Compound No. | Yield (%) | Melting point (°C) | $^1$H-NMR spectrum ($CDCl_3$ solution, δ ppm) |
|---|---|---|---|
| 174 | 66 | 103-105 | 0.96(3H, d, J=7.0Hz), 1.10(3H, m), 1.65(2H, m), 2.85(2H, m), 3.19(3H, s), 3.40(2H, s), 4.72(2H, br.d, J=12.6Hz), 7.90 (1H, s). |
| 176 | 52 | 140-141 | 0.92(3H, s), 0.99(3H, s), 1.65(4H, m), 2.31(2H, d.d, J=10.5, 13.3Hz), 3.20(3H, s), 3.40(2H, s), 4.72(2H, br.d, J=12.6Hz), 7.90(1H, s). |
| 544 | 40 | 114-115 | 0.90(6H, d, J=7Hz), 1.1-3.0(10H, m), 3.19(3H, s), 3.39(2H, s), 4.80(2H, m), 7.86(1H, s). |
| 548 | 36 | 123-127 | 0.90(9H, s), 1.1-1.9(5H, m), 2.76(2H, m), 3.21(3H, s), 3.41 (2H, s), 4.85(2H, m), 7.90(1H, s). |
| 552 | 88 | 85-90 | 1.4-2.1(4H, m), 2.6-3.4(3H, m), 3.22(3H, s), 3.44(2H, s), 4.95(2H, br.d, J=12.6Hz), 7.27(5H, s), 7.95(1H, s). |
| 556 | 79 | 115-118 | 1.0-3.0(11H, m), 3.15(3H, s), 3.37(2H, s), 4.72(2H, m), 7.16 (5H, m), 7.84(1H, s). |
| 558 | 43 | 67-71 | 1.4-2.2(4H, m), 3.0-3.6(3H, m), 3.18(3H, s), 3.40(2H, s), 4.50(1H, t),4.64(1H, t), 7.2-7.5(5H, m), 7.86(1H, s). |
| 560 | 71 | — | 1.94(4H, m), 2.90(1H, m), 3.20(3H, s), 3.43(2H, s), 3.5-4.3 (4H, m), 7.92(1H, s). |

- to be continued -

| Compound No. | Yield (%) | Melting point (°C) | $^1H$-NMR spectrum ($CDCl_3$ solution, δ ppm) |
|---|---|---|---|
| 564 | 67 | 84-87 | 1.27(3H, t, J=7Hz), 1.5-3.1(7H, m), 3.21(3H, s), 3.42(2H, s), 4.16(2H, q, J=7Hz), 4.67(2H, m), 7.91(1H, s). |
| 568 | 61 | 130-132 | 1.20-3.04(17H, m), 3.21(3H, s), 3.41(2H, s), 4.85(2H, m), 7.91(1H, s). |
| 572 | 68 | — | 2.94(2H, t, J=7Hz), 3.25(3H, s), 3.43(2H, s), 4.08(2H, t, J=7Hz), 4.93(2H, s), 7.21(4H, m), 7.96(1H, s). |
| 576 | 34 | — | 2.85(2H, t, J=7Hz), 3.24(3H, s), 3.42(2H, s), 3.87(6H, s), 4.06(2, t, J=7Hz), 4.85(2H, s), 6.68(2H, m), 7.95(1H, s). |
| 580 | 44 | 123-125 | 2.90(2H, t, J=7Hz), 3.11(3H, s), 3.21(3H, s), 3.41(2H, s), 3.86(2H, t, J=7Hz), 7.26(5H, m), 7.93(1H, s). |

- to be continued -

EXAMPLE 2

Ethyl 2-morpholino-4-diethylaminopyrimidine-5-acetate (compound No. 404):-

Phosphorus oxychloride (11.7 ml) was added to 3.35 g (12.5 mmoles) of ethyl 2-morpholino-4-hydroxypyrimidine-5-acetate, and the mixture was heated under reflux for 4 hours. The reaction mixture was concentrated under reduced pressure, and methylene chloride and ice water were added. It was neutralized with sodium hydrogen carbonate. The methylene chloride layer was dried over anhydrous sodium sulfate, and concentrated under reduced pressure. To the product were added 15 ml of ethanol and 7.0 g (95.7 mmoles) of diethylamine, and in an autoclave, the mixture was reacted at 120 °C for 7 hours. Water was added, and the mixture was extracted with methylene chloride. The solvent was evaporated, and the residue was purified by silica gel column chromatography to give 2.8 g (yield 56 %) of the desired compound as an oil.

$^1$H-NMR spectrum ($CDCl_3$ solution, δppm): 1.16(6H, t, J = 7Hz), 1.24(3H, t, J = 7Hz), 3.20(4H, q, J = 7Hz), 3.44(2H, s), 3.74 (8H, s), 4.16(2H, q, J = 7Hz), 7.80(1H, s).

EXAMPLE 3

2-Methylthio-5,6-dihydro-7-methyl-6-oxo(7H)pyrrolo[2,3-d]pyrimidine (compound No. 134):-

Phosphorus oxychloride (59.0 g) was added to 4.84 g (21.2 mmoles) of ethyl 2-methylthio-4-hydroxypyrimidine-5-acetate, and the mixture was heated under reflux for 3 hours. The reaction mixture was concentrated under reduced pressure, and after adding chloroform, neutralized with an aqueous solution of sodium hydrogen carbonate. The chloroform layer was concentrated under reduced pressure. To the concentrate were added 20 ml of ethanol and 3.48 g (44.9 mmoles) of a 40 % methanol solution of methylamine, and the mixture was reacted at 100 °C for 5 hours in an autoclave. The solvent was evaporated under reduced pressure, and after adding water, the mixture was extracted with chloroform. The chloroform layer was concentrated under reduced pressure, treated with activated carbon in ethanol, and recrystallized to give 1.50 g (yield 36 %) of the desired compound.

Melting point: 183-185 °C (decomp.)

$^1$H-NMR spectrum ($CDCl_3$ solution, δppm): 2.60(3H, s), 3.28(3H, s), 2.54(2H, s), 8.14(1H, s).

EXAMPLE 4

2-iso-Propylamino-5,6-dihydro-7-methyl-6-oxo(7H)pyrrolo(2,3-d]pyrimidine hydrochloride (compound No. 102):-

0.49 g (2.4 mmoles) of 2-isopropylamino-5,6-dihydro-7-methyl-6-oxo(7H)pyrrolo[2,3-d]pyrimidine was dissolved in 30 ml of toluene, and hydrogen chloride gas was blown into the solution. The solution was concentrated under reduced pressure, and then washed with hexane to give 0.53 g (yield 92 %) of the desired compound.

Melting point: 272-275 °C

$^1$H-NMR spectrum ($CDCl_3$ solution, δppm): 1.38(6H, d, J = 5Hz), 3.30(3H, s), 3.60 (2H, br.s), 4.30(1H, br.), 7.90(1H, br.), 8.90(1H, br.).

Similarly, compounds tabulated below were produced.

| Compound No. | Yield (%) | Melting point (°C) | $^1$H-NMR spectrum ($CDCl_3$ solution, δ ppm) |
|---|---|---|---|
| 116 | 93 | 203-205 (decomp.) | 1.79(6H, br.s), 3.31(3H, s), 3.62(2H, s), 4.04(4H, br.s), 8.04(1H, br.s). |
| 122 | 100 | 251-253 | 3.27(3H, s), 3.64(2H, s), 3.84(4H, m), 4.10(4H, m), 8.06 (1H, s). |
| 126 | 91 | 239-241 (decomp.) | 2.84(4H, m), 3.28(3H, s), 3.65(2H, s), 4.38(4H, m), 8.12 (1H, s). |
| 129* | 91 | | 2.1-2.3(2H, m), 3.20(3H, s), 3.2-3.4(4H, m), 3.66(2H, s), 3.9-4.1(2H, m), 4.1-4.3(2H, m), 8.02(1H, s). |
| 502 | 100 | deliquescent 213-214 | 1.10(3H, br.s), 1.80(5H, m), 2.4-3.4(2H, m), 3.30(3H, s), 3.60(2H, s), 4.76(1H, br.d, J=12.6Hz), 8.08(1H, s). |
| 175 | 40 | deliquescent 186-190 | 1.0(3H, d. J=7.0Hz), 2.0-2.1(5H, m), 2.9-3.5(2H, m), 3.25(3H, s), 3.59(2H, s), 4.85(2H, br.d, J=12.6Hz), 8.05 (1H, br.s). |
| 177 | 100 | 238-240 | 1.05(6H, m), 1.4-2.1(4H, m), 2.2-2.9(2H, m), 3.28(3H, s), 3.61(2H, s), 4.90(2H, br.d, J=12.6Hz), 8.06(1H, s). |

- to be continued -

| Compound No. | Yield (%) | Melting point (°C) | $^1$H-NMR spectrum ($CDCl_3$ solution, δ ppm) |
|---|---|---|---|
| 546 | 88 | 240-242 (decomp.) | 0.90(6H, d, J=7Hz), 1.0-2.2(7H, m), 2.8-3.7(8H, m), 4.92 (2H, m), 8.0(1H, s). |
| 550 | 82 | 234-236 (decomp.) | 0.90(9H, s), 1.35(3H, m), 1.94(2H, m), 3.10(2H, m), 3.27 (3H, s), 3.58(2H, s), 5.00(2H, m), 8.05(1H, s). |
| 554 | 90 | 138-140 | 1.5-2.4(4H, m), 2.6-3.5(3H, m), 3.28(3H, s), 3.61(2H, s), 5.10(2H, br.d, J=12.6Hz), 7.25(5H, m), 8.10(1H, s). |
| 557 | 83 | 212-215 (decomp.) | 1.1-3.2(11H, m), 3.24(3H, s), 3.59(2H, s), 4.90(2H, m), 7.20 (5H, m), 8.03(1H, s). |
| 562 | 90 | 173-175 (decomp.) | ($CDCl_3$-$CD_3OD$) 2.10(4H, m), 3.12(1H, m), 3.30(3H, s), 3.65(2H, s), 4.14 (4H, m), 7.94(1H, s). |
| 566 | 85 | 196-198 (decomp.) | 1.30(3H, t. J=7Hz), 1.6-2.9(5H, m), 3.30(3H, s), 3.49(2H, s), 3.65(2H, m), 4.18(2H, q, J=7Hz), 4.70(2H, m), 8.10(1H, s). (1H, br.s). |
| 570 | 71 | 270-275 (decomp.) | 1.60-3.06(13H, m), 3.22(3H, s), 3.48(2H, s), 3.52(4H, m), 5.03(2H, m), 7.94(1H, s). |
| 574 | 82 | 231-237 (decomp.) | 3.10(2H, m), 3.33(3H, s), 3.63(2H, s), 4.24(2H, m), 5.10 (2H, m), 7.26(4H, m), 8.13(1H, s). |

- to be continued -

| Compound No. | Yield (%) | Melting point (°C) | $^1$H-NMR spectrum ($CDCl_3$ solution, δ ppm) |
|---|---|---|---|
| 578 | 82 | 223-227 (decomp.) | 3.0(2H, m), 3.35(3H, s), 3.65(2H, s), 3.91(6H, s), 4.04 (2H, m), 4.98(2H, s), 6.80(2H, m), 8.10(1H, s). |
| 582 | 95 | 255-258 | ($CDCl_3$-$CD_3OD$) 3.03(2H, t, J=7Hz), 3.27(3H, s), 3.32(3H, s), 3.64(2H, s), 4.07(2H, t, J=7Hz), 7.29(5H, m), 7.95(1H, s). |
| 559 | 95 | 106-110 | 1.4-2.3(4H, m), 3.0-3.8(3H, m), 3.22(3H, s), 3.58 (2H, s), 4.3-4.8(br. 2H), 7.1-74.(5H, m), 7.99(1H, s). |
| 211* | 100 | 151-153 | 0.88(3H, t, J=7Hz), 1.52(2H, m), 2.98(3H, s), 3.22(2H, m), 4.34(2H, s), 8.50(1H, s). |

- to be continued -

| Compound No. | Yield (%) | Melting point (°C) | $^1$H-NMR spectrum ($CDCl_3$ solution, δ ppm) |
|---|---|---|---|
| 239 | 100 | >300 | 1.46(6H, s), 1.48(6H, s), 1.70(8H, m), 2.99(3H, s), 4.33 (3H, br.s), 8.08(2H, m), 8.56(1H, s). |
| 241 | 100 | 278-281 | 1.70(6H, m), 3.0(3H, s), 3.25(6H, m), 3.75(2H, m), 4.35 (2H, s), 8.10(1H, m), 8.59(1H, s). |

* $^1$H-NMR was measured in DMSO-$d^6$ solution.

EXAMPLE 5

2-iso-Propylamino-5,6-dihydro-7-methyl-6-oxo(7H)pyrrolo(2,3-d]pyrimidine maleate (compound No. 104):-

6.37 g (30.9 mmles) of 2-isopropylamino-5,6-dihydro-7-methyl-6-oxo(7H)pyrrolo[2,3-d]pyrimidine was dissolved in 50 ml of ethyl acetate, and 3.58 g (30.8 mmoles) of maleic acid was added. The mixture was stirred at room temperature for 1 hour. The resulting crystals were collected by filtration to give 8.90 g (yield 90 %) of the desired compound.

Melting point: 158-160 °C.

$^1$H-NMR spectrum ($CDCl_3$ solution, δppm): 1.24(6H, d, J=6Hz), 3.12(3H, s), 3.50 (2H, s), 3.9-4.2(1H, m), 6.20(2H, s), 7.90(1H, s).

Similarly, compounds tabulated below were produced.

| Compound No. | Yield (%) | Melting point (°C) | $^1$H-NMR spectrum ($CDCl_3$ solution, δ ppm) |
|---|---|---|---|
| 108 | 89 | 155-162 (decomp.) | 3.20(3H, s), 3.26(6H, s), 3.50(2H, s), 6.20(2H, s), 7.94 (1H, s). |
| 112** | 79 | 128-130 | 2.0-2.2(4H, m), 3.22(3H, s), 3.56(2H, br.s), 3.6-3.8(4H, m), 6.24(4H, s), 7.90(1H, s). |
| 118 | 69 | 135-137 | 1.64(6H, br.s), 3.12(3H, s), 3.46(2H, s), 3.7-3.9(4H, m), 6.22(2H, s), 7.90(1H, s). |
| 406 | 56 | oil | 1.28(6H, t, J=7Hz), 1.30(3H, t, J=7Hz), 3.54(2H, s), 3.60 (4H, q, J=7Hz), 3.80(8H, s), 4.22(2H, q, J=7Hz), 6.32(2H, s), 7.89(1H, s), 10.91(2H, br.s). |

** dimaleate

EXAMPLE 6

2-iso-Propylamino-5-oxo-6-methyl-5,6-dihydro(7H)pyrrolo[3,4-d]pyrimidine (compound No. 202):-

A solution composed of 2.2 g (32 mmoles) of isopropylamine, 5.18 g (37 mmoles) of s-methylisothiourea sulfate and 20 ml of water was stirred at room temperature for 24 hours. Water was evaporated under reduced pressure. To the residue were added 7.8 g (35 mmoles) of ethyl 4-chloro-2-ethoxymethyleneacetoacetate and 30 ml of methanol and further 1.4 g of sodium hydroxide. The mixture was stirred for 2 hours, and then 27 g (348 mmoles) of a 40 % methanol solution of methylamine was added dropwise. After the addition, the mixture was further stirred for 2 hours. The precipitated crystals were collected by filtration, and extracted with water and chloroform. The chloroform layer was dried over anhydrous magnesium sulfate. The solvent was evaporated under reduced pressure to give 0.8 g (yield 12 %) of the desired compound.

Melting point: 201-202 °C

$^1$H-NMR spectrum ($CDCl_3$ solution, δppm): 1.27(6H, d, J=7Hz), 3.12(3H, s), 4.20 (2H, s), 4.27(1H, m), 5.50(1H, br.s), 8.63(1H, s).

EXAMPLE 7

2-Morpholino-6-methyl-5-oxo-5,6-dihydro(7H)pyrrolo[3,4-d]pyrimidine (compound No. 232):-

79 g (320 mmoles) of 4-chloromethyl-5-ethoxycarbonyl-2-methylthiopyrimidinewas dissolved in 300 ml of methanol, and 50 g (640 mmoles) of a 40 % methanol solution of methylamine was added dropwise over 15 minutes, and the mixture was stirred for 15 hours. The product was separated by filtration and dried to give 11 g (yield 18 %) of 2-methylthio-6-methyl-5-oxo-5,6-dihydro(7H)pyrrolo[3,4-d]pyrimidine. The resulting product (1.5 g; 7.7 mmoles) and 3.4 g (38.5 mmoles) of morpholine were dissolved in 20 ml of n-amyl alcohol, and the solution was heated under reflux for 7 hours. The reaction mixture was cooled, and the precipitated crystals were separated by filtration to give 0.75 g (yield 42 %) of the desired compounds.

Melting point: 184-187 °C (decomp.)

$^1$H-NMR spectrum ($CDCl_3$ solution, δppm): 3.16(3H, s), 3.85(8H, m), 4.24(2H, s), 8.68(1H, s).

By the same way, the following compounds were prepared.

| Com-pound No. | Yield (%) | Melting point (°C) | $^1$H-NMR spectrum ($CDCl_3$ solution, δ ppm) |
|---|---|---|---|
| 214 | 36 | 149-151.3 | 1.00(6H, d, J=7Hz), 1.93(1H, m), 3.14(3H, s), 3.34(2H, t, J=7Hz), 4.23(2H, s), 8.62(1H, s). |
| 216 | 64 | 204-205.5 | 2.03(4H, m), 3.03(3H, s), 3.65(4H, m), 4.23(2H, s), 8.67 (1H, s). |
| 218 | 37 | 175.5-177 | 1.64(6H, m), 3.14(3H, s), 3.87(4H, m), 4.19(2H, s), 8.64 (1H, s). |
| 226 | 28 | 133.5-135.5 | 1.67(8H, m), 3.12(3H, s), 3.82(4H, t, J=7Hz), 4.20(2H, s), 8.65(1H, s). |
| 236 | 55 | 169-170 | 2.96(4H, m), 3.15(3H, s), 4.24(2H, s), 4.26(4H, m), 8.67 (1H, s). |
| 210 | 51 | — | 1.00(3H, t, J=7Hz), 1.66(2H, sex, J=7Hz), 3.15(3H, s), 3.46 (2H, q, J=7Hz), 4.24(2H, s), 8.64(1H, s). |
| 584 | 63 | 114-116 | 0.98(6H, d, J=7Hz), 1.4-1.9(4H, m), 2.40(2H, t, J=12.6Hz), 3.13(3H, s), 4.20(2H, s), 4.86(2H, br.d, J=12.6Hz), 8.64 (1H, s). |

- to be continued -

| Compound No. | Yield (%) | Melting point (°C) | $^{1}H$-NMR spectrum ($CDCl_3$ solution, δ ppm) |
|---|---|---|---|
| 234 | 57 | 221-222 | 1.25(3H, s), 1.32(3H, s), 2.67(2H, d.d, J=10.8, 14.2Hz), 3.14 (3H, s), 3.65(2H, m), 4.22(2H, s), 4.70(2H, d.d, J=10.8, 1.5Hz), 8.68(1H, s). |
| 238 | 29 | 139-140 | 1.01(2H, d.d, J=12.3, 12.3Hz), 1.18(6H, s), 1.32(6H, s), 2.04(2H, d.d, J=12.3, 3.6Hz), 3.15(3H, s), 4.24(2H, s), 4.40 (1H, m), 5.36(1H, br.d, J=7.2Hz), 8.65(1H, s). |
| 240 | 46 | 131-132 | 1.52(6H, m), 2.44(4H, m), 2.56(2H, t, J=7.2Hz), 3.15(3H, s), 3.56(2H, dt, J=7.2, 5.4Hz), 4.23(2H, s), 6.27(1H, m), 8.66 (1H, s). |

EXAMPLE 8

Methyl 2-piperidino-4-aminopyrimidine-5-carboxylate (compound No. 400):-

Ethylene dichloride (50 ml) and 10 ml of phosphorus oxychloride were added to 5.6 g (23.6 mmoles) of methyl 2-piperidino-4-hydroxypyrimidine-5-carboxylate, and the mixture was heated under reflux for 5.5 hours. The reaction mixture was concentrated under reduced pressure, and after adding chloroform and water, neutralized with sodium hydrogen carbonate. The chloroform layer was separated and the solvent was evaporated. To the residue were added 70 ml of THF and 27.8 g of 25 % ammonium hydroxide. The mixture was reacted at 70 °C for 1.5 hours in an autoclave. The reaction mixture was concentrated under reduced pressure and recrystallized from toluene/hexane to give 5.0 g (yield 90 %) of the desired compound.

$^1$H-NMR spectrum ($CDCl_3$ solution, δppm): 1.62(6H, m), 3.82(3H, s), 3.80(4H, m), 8.60(1H, s).

EXAMPLE 9

2-iso-Propylamino-5-oxo-6-methyl-5,6-dihydro(7H)pyrrolo[3,4-d]pyrimidine hydrochloride (compound No. 204):-

Concentrated hydrochloric acid (0.27 g; 2.7 mmoles) was added to a solution of 0.56 g (2.7 mmoles) of 2-isopropylamino-5-oxo-6-methyl-5,6-dihydro(7H)pyrrolo[3,4-d]pyrimidine in 6 ml of chloroform, and the solution was stirred for 30 minutes. The solvent was then evaporated under reduced pressure, and the residue was washed with ether to give 0.6 g (yield 92 %) of the desired compound.

Melting point: 176-183 °C (decomp.)

Similarly, the following compounds were produced.

| Compound No. | Yield (%) | Melting point (°C) | $^1$H-NMR spectrum ($CDCl_3$ solution, δ ppm) |
|---|---|---|---|
| 215 | 100 | 167-168 | 0.94(6H, d, J=7Hz), 1.86(1H, m), 2.97(3H, s), 3.20(2H, m), 3.37(2H, s), 8.57(1H, s). |
| 402 | 100 | >300 | 1.62(6H, br.s), 3.80(7H, br.s), 8.30(1H, s). |

EXAMPLE 1B

Tablets each containing 10 mg of active ingredient were prepared by the following procedure.

| | Per tablet |
|---|---|
| Active ingredient | 10 mg |
| Corn starch | 55 mg |
| Crystalline cellulose | 35 mg |
| Polyvinyl pyrrolidone (as 10 % aqueous solution) | 5 mg |
| Carboxymethyl cellulose calcium | 10 mg |
| Magnesium stearate | 4 mg |
| Talc | 1 mg |
| Total | 120 mg |

The active ingredient, corn starch and crystalline cellulose were passed through an 80-mesh sieve and thoroughly mixed. The mixed powder was granulated together with the polyvinyl pyrrolidone solution, and passed through an 18-mesh sieve. The resulting granules were dried at 50 to 60 °C and again passed through an 18-mesh sieve to adjust their sizes. The carboxymethyl cellulose calcium, magnesium stearate and talc, which had been passed through an 80-mesh sieve, were added to the granules. They were mixed and tableted by a tableting machine to produce tablets each having a weight of 120 mg.

EXAMPLE 2B

Tablets each containing 200 mg of active ingredient were produced by the following procedure.

| | Per tablet |
|---|---|
| Active ingredient | 200 mg |
| Corn starch | 50 mg |
| Crystalline cellulose | 42 mg |
| Silicic anhydride | 7 mg |
| Magnesium stearate | 1 mg |
| Total | 300 mg |

The above components were passed through an 80-mesh sieve and thoroughly mixed. The resulting mixed powder was compression-molded to produce tablets each having a weight of 300 mg.

EXAMPLE 3B

Capsules each containing 100 mg of active ingredient were produced by the following procedure.

| | Per capsule |
|---|---|
| Acive ingredient | 100 mg |
| Corn starch | 40 mg |
| Lactose | 5 mg |
| Magnesium stearate | 5 mg |
| Total | 150 mg |

The above components were mixed, passed through an 80-mesh sieve, and thoroughly mixed. The resulting mixed powder was filled into capsules in an amount of 150 mg for each.

EXAMPLE 4B

Injectable preparations in vials each containing 5 mg of active ingredient were produced by the following procedure.

| | Per vial |
|---|---|
| Active ingredient<br>Mannitol | 5 mg<br>50 mg |

Just prior to use, these compounds were dissolved in 1 ml of distilled water for injection, and administered.

EXAMPLE 5B

Injectable preparations in ampoules each containing 50 mg of active ingredients were produced in accordance with the following recipe.

| | Per ampoule |
|---|---|
| Active ingredient<br>Sodium chloride<br>Distilled water for injection | 50 mg<br>18 mg<br>proper amount |
| Total | 2 ml |

EXAMPLE 6B

An adhesive patch containing 17.5 mg of active ingredient was produced by the following procedure.

Ten parts of poly(ammonium acrylate) was dissolved in 60 parts of water. Two parts of glycerin diglycidyl ether was dissolved under heat in 10 parts of water. Furthermore, 10 parts of polyethylene glycol (grade 400), 10 parts of water and 1 part of an active ingredient were stirred to form a solution. While the aqueous solution of poly(ammonium acrylate) was stirred, the aqueous solution of glycerin diglycidiyl ether and the solution containing the active ingredient, polyethylene glycol and water were added and mixed. The resulting solution for hydrogel was coated on a pliable plastic film so that the rate of the active ingredient was 0.5 mg per $cm^2$. The surface was covered with releasing paper and cut to a size of 35 $cm^2$ to form an adhesive patch.

EXAMPLE 7B

An adhesive patch containing 10 mg of active ingredient was produced by the following procedure.

An aqueous sol is prepared from 100 parts of poly(sodium acrylate), 100 parts of glycerin, 150 parts of water, 0.2 part of triepoxypropyl isocyanurate, 100 parts of ethanol, 25 parts of isopropyl myristate, 25 parts of propylene glycol and 15 parts of the active ingredient. The sol was then coated to a thickness of 100 micrometers on the non-woven fabric surface of a composite film composed of a rayon non-woven fabric and a polyethylene film to form an adhesive layer containing the drug. The amount of the release aids (isopropyl myristate and propylene glycol) contained in this layer was about 30 % by weight. The adhesive layer was then crosslinked at 25 °C for 24 hours, and a releasing film was bonded to the adhesive layer surface. The entire film was then cut into pieces each having an area of 35 $cm^2$.

The biological activity in vitro of the active ingredient on cells of the nervous system were tested. The cells tested were mouse neuroblastoma cell line neuro-2a (Dainippon Pharmaceutical Co., Ltd.) which have been established as the cells of the nervous system. The above nerve cells were grown in an incubator at 37 °C in the presence of 5% carbon dioxide gas exponentially, and then cultivated for a certain period of time together with the active ingredient. The results demonstrate that the active ingredient has nerve cell growth promoting activity and neurite formation and sprouting promoting activity which are markedly higher and significantly than a control, and are equal to, or higher than, isaxonine as a control drug (the compound described in JP-B-28548/1984).

The biological activity of the active ingredient on a rat PC-12 pheochromocytoma cell line was also tested. when NGF is added to P-12 cells, the neurites sprout. It was shown that when the active ingredient is added at this time, the binding of NGF to the PC-12 cells and the up-take of NGF into the cells increased.

When the effect of the active ingredient on the binding of NGF to rabbit superior cervical ganglion was examined, it was found to promote the NGF binding.

Rats whose sciatic nerves were crushed were prepared as a model of peripheral nervous disorder, and the effects of active ingredient on it were tested. It was clear that the active ingredient promoted the recovery of the interdigit distance and the weight of the soleus muscle to normal values.

Rat and mouse models of central nervous disorders were prepared, and the pharmacological effects of the active ingredient was tested. Specifically, nigral dopamine cells of the rat brain were chemically destroyed by injecting a very small amount of 6-hydroxydopamine to induce motor imbalance. Two weeks later, dopamine cells of fetal brain were transplanted in the caudate nucleus into the lesioned side of the rat brain and an attempt was made to improve motor imbalance. Specifically, beginning on the day of transplantation, the active ingredient was intraperitoneally administered every day over 2 weeks, and it's activity in improving motor imbalance and the growth of the transplanted cells were examined. It was found that the active ingredient has promoted improvement in motor trouble.

Rats and mice having nerve trouble caused by mercury poisoning were prepared and the activity of the active ingredient was tested. The active ingredient was found to promote an improvement in the condition and recovery to normal, a curative effect on chemical-induced disorders and an effect of improving and recovering learning and memory.

Thus, it has been made clear that the active ingredient is useful as an agent for improving or curing various neurological diseases of mammals, such as disorders of the peripheral and central nerves, and also as an agent for improving learning and memory.

Various types of neuropathy including, for example, various peripheral nerve disorders accompanied by motorgenic, sensory or objective flex retardation, and alcohol-induced or drug-induced, diabetic and metabolic, or idiopathic peripheral nerve disorders, including traumatic, inflammatory or immunological nerve root lesions may be cited as such neurological diseases. More specific examples include facial palsy, sciatic nerve paralysis, spinal muscular atrophy, muscular dystrophy, myasthenia gravis, multiple sclerosis, amyotrophic lateral sclerosis, acute disseminated cerebromyelitis, Guillan-Barre syndrome, postvaccinal encephalomyelitis, SMON disease, dementia, Alzheimer syndrome, a condition after cranial injury, cerebral ischemia, sequela of cerebral infarction of cerebral hemorrhage, and rheumatism. These examples are not limitative.

By a toxicity test, the compounds of this invention were found to have only weak toxicity and side effects, and can be used as safe and highly useful medicines.

EXPERIMENTAL EXAMPLE 1

The effects of the compounds of this invention on neuroblastoma cells were examined by the following method. Mouse neuro 2a cells in the logarithmic growth period in the Dulbecco's modified Eagle's medium [DMEM, containing 100 units/ml of penicillin G sodium and 100 micrograms/ml of streptomycin sulfate] containing 10 % of FCS were seeded in a 48-well plate so that the number of cells was 1,000 cells/well, and cultured for one day in 0.25 ml of the culture fluid in each well in an incubator containing 5 % of carbon dioxide gas in air at 37 °C. Then, a 4 % aqueous glutaraldehyde solution in the same amount as a medium (0.25 ml) was added, and the culture fluid was left to stand at room temperature for 2 hours to fix the cells. After washing with water, a 0.05 % aqueous solution of methylene blue was added to stain the cells. Under a microscope, the number of cells containing outgrown neurites (cells having at least one neurite with a length of at least two times as large as the long diameter of the cell) was counted visually, and the proportion of these cells in the entire cells was calculated. The well was observed over 5 or more visual fields (at least 2 % of the entire surface area of the well) continuous to the left and right from a mark put at the center of the well, and more than 200 cells were counted. One drug compound was used in 6 different concentrations at most, and three runs were conducted for each concentration. The results were expressed as a mean ±S.D., and the results are shown in Table 1.

Mouse neuroblastoma cells NS-20Y were similarly cultured in a dish coated with polyornithine, and the effects of the compounds were examined. The results obtained after 24 hours and 48 hours from the start of culturing are shown in Table 2.

Table 1

Action on neuro-2-a cells

| Run No. | Compound | Number of cells having neurites with a length at least two times the diameter of cells/total number of cells, % (concentration of the compound) |
|---|---|---|
| 1 | 402 | 29.6±5.5(3mM), 25.9±3.6(10mM), 24.6±6.3(1mM), 18.9±2.5(20mM), 11.9±5.0(0.3mM), 5.3±0.8(0.1mM). |
| | isaxonine | 10.9±1.7(3mM). |
| | control | 1.9±0.9 |
| 2 | 128 | 39.4±1.9(1mM), 16.1±2.5(0.3mM), 6.4±1.5(0.1mM). |
| | 112 | 20.9±1.3(3mM), 10.2±1.6(1mM), 4.7±0.4(0.3mM). |
| | isaxonine | 32.7±1.7(10mM). |
| | control | 1.8±0.9 |
| 3 | 102 | 30.5±0.3(3mM), 15.1±2.0(1mM), 5.3±1.3(0.3mM). |
| | isaxonine | 28.5±3.0(10mM). |
| | control | 2.5±0.7 |
| 4 | 204 | 22.8±1.1(10mM), 20.1±5.1(5mM), 9.4±1.7(3mM). |
| | control | 2.0±0.7 |

- to be continued -

Table 1 (continued)

| Run No. | Compound | Number of cells having neurites with a length at least two times the diameter of cells/total number of cells, % (concentration of the compound) |
|---|---|---|
| 5 | 104 | 28.4±1.4(3mM), 12.3±3.3(1mM), 7.2±0.7(0.3mM), 4.6±0.7(0.03mM). |
| | 118 | 21.0±1.8(1mM), 7.6±1.0(0.3mM), 4.8±0.3(0.03mM). |
| | 108 | 14.4±1.3(3mM), 5.7±1.1(1mM), 3.9±1.6(0.1mM), 3.0±1.0(0.03mM). |
| | isaxonine | 32.7±4.4(10mM), 8.0±1.5(20mM), 8.0±1.2(3mM). |
| | control | 1.8±0.8 |
| 6 | 122 | 15.7±1.3(3mM), 4.4±1.1(0.1mM), 4.0±1.2(1mM). |
| | 406 | 12.9±3.7(1mM), 10.4±1.0(0.3mM), 5.2±1.7(0.03mM). |
| | 216 | 6.7±0.9(3mM), 6.5±3.3(10mM). |
| | 226 | 8.1±3.4(1mM), 4.6±0.9(0.3mM). |
| | 126 | 24.7±0.7(10mM), 14.9±0.9(3mM), 9.2±1.7(1mM). |
| | 218 | 9.9±2.2(3mM), 5.0±1.3(1mM), |
| | isaxonine | 32.9±3.5(10mM), 7.6±2.7(3mM). |
| | control | 2.8±0.4 |

- to be continued -

Table 1 (continued)

| Run No. | Compound | Number of cells having neurites with a length at least two times the diameter of cells/total number of cells, % (concentration of the compound) |
|---|---|---|
| 7 | 502 | 4.1±0.6(0.1mM), 7.5±0.2(0.2mM), 11.0±4.8(0.3mM), 20.7±2.8(0.5mM). |
| | 175 | 4.2±0.8(0.1mM), 11.7±1.3(0.2mM), 21.0±1.4(0.3mM), 15.7±1.7(0.5mM). |
| | 554 | 7.3±0.9(0.1mM), 30.7±1.0(0.2mM), 34.0±2.9(0.3mM), 22.0±6.1(0.5mM). |
| | isaxonine | 27.8±1.1(10mM). |
| | control | 2.5±0.1 |
| 8 | 177 | 5.0±3.0(0.1mM), 15.7±4.9(0.2mM), 27.2±1.5(0.3mM), 16.3±1.8(0.5mM). |
| | isaxonine | 22.2±3.1(5mM). |
| | control | 1.7±0.3 |
| 9 | 550 | 3.1±1.0(0.01mM), 3.6±1.4(0.03mM), 36.1±0.4(0.1mM), 14.3±5.9(0.3mM). |
| | 562 | 5.2±1.5(0.3mM), 5.8±1.7(1mM), 10.2±2.6(3mM), 12.5±0.4(10mM). |
| | isaxonine | 30.2±3.5(10mM). |
| | control | 2.6±1.0 |

- to be continued -

Table 1 (continued)

| Run No. | Compound | Number of cells having neurites with a length at least two times the diameter of cells/total number of cells, % (concentration of the compound) |
|---|---|---|
| 10 | 566 | 7.5±3.0(0.3mM), 5.4±2.6(1mM). |
| | isaxonine | 15.7±4.1(3mM). |
| | control | 1.2±1.1 |
| 11 | 574 | 12.1±0.6(0.3mM), 11.6±3.3(1mM). |
| | 578 | 6.3±1.7(0.03mM), 6.6±3.0(0.1mM). |
| | isaxonine | 26.7±7.7(10mM). |
| | control | 1.8±0.8 |
| 12 | 582 | 7.9±0.8(0.1mM), 9.8±2.0(0.3mM), 24.1±8.6(1mM), 12.8±2.8(3mM). |
| | isoxonine | 30.8±2.9(10mM). |
| | control | 3.2±1.6 |

- to be continued -

Table 1 (continued)

| Run No. | Compound | Number of cells having neurites with a length at least two times the diameter of cells/total number of cells, % (concentration of the compound) |
|---|---|---|
| 13 | 570 | 10.6±1.9(0.03mM), 17.1±0.6(0.1mM), 29.4±6.8(0.3mM), 8.7±0.8(1mM). |
| | isaxonine | 30.7±5.9(10mM). |
| | control | 2.9±1.9 |
| 14 | 546 | 6.4±1.0(0.03mM), 16.3±1.2(0.1mM), 26.9±4.8(0.3mM), 46.3±5.5(1mM). |
| | 557 | 4.3±1.7(0.03mM), 25.6±3.9(0.1mM). |
| | isaxonine | 17.4±4.2(3mM), 23.3±2.2(10mM). |
| | control | 2.3±0.6 |

- to be continued -

Table 1 (continued)

| Run No. | Compound | Number of cells having neurites with a length at least two times the diameter of cells/total number of cells, % (concentration of the compound) |
|---|---|---|
| 15 | 215 | 5.8±0.3(0.3mM), 14.5±2.4(3mM). |
| | 232 | 5.3±3.1(1mM), 8.9±0.5(3mM). |
| | 236 | 4.2±0.6(0.3mM), 6.2±0.5(1mM). |
| | 234 | 5.5±1.7(3mM), 8.9±0.9(10mM). |
| | 239 | 3.4±1.6(0.03mM), 3.4±0.9(0.1mM). |
| | isaxonine | 22.1±2.1(10mM), 10.5±4.9(3mM). |
| | control | 2.4±0.2 |
| 16 | 175 | 6.1±1.0(0.1mM), 27.9±4.4(0.3mM). |
| | isaxonine | 27.0±3.8(10mM). |
| | control | 3.3±0.4 |
| 17 | 502 | 6.9±1.7(0.1mM), 12.2±2.0(0.3mM). |
| | isaxonine | 25.6±6.2(10mM). |
| | control | 2.2±0.5 |

- to be continued -

Table 1 (continued)

| Run No. | Compound | Number of cells having neurites with a length at least two times the diameter of cells/total number of cells, % (concentration of the compound) |
|---|---|---|
| 18 | 211 | 5.4±0.7(0.1mM), 5.3±0.2(1mM), 19.0±2.9(3mM). |
| | 584 | 4.4±1.8(0.03mM), 4.2±0.6(0.3mM). |
| | 241 | 5.6±2.7(0.3mM), 10.0±0.7(1mM). |
| | 177 | 4.9±2.4(0.03mM), 6.1±0.1(0.1mM), 14.2±1.1(0.3mM), 28.4±4.5(1mM). |
| | 554 | 4.6±2.7(0.03mM), 9.6±2.7(0.1mM), 20.2±2.2(0.3mM), 35.8±9.8(1mM). |
| | isaxonine | 21.0±1.4(10mM), 9.6±1.7(3mM). |
| | control | 3.3±0.4 |
| 19 | 559 | 9.2±0.8(0.1mM). |
| | isaxonine | 19.4±3.1(10mM). |
| | control | 2.4±0.9 |

Table 2

Activity on NS-20Y cells

| Compound | Number of cells in which neurites appeared/total number of cells (concentration of the compound) | |
|---|---|---|
| 118 | 35/50(1.0mM)<br>4/52(0.5mM) | 25/50(1.0mM)<br>9/49(0.5mM) |
| control | 1/56 | 1/52 |
| 122 | 4/54(1.0mM)<br>1/52(0.5mM) | 10/52(0.5mM)<br>8/52(0.3mM) |
| control | 1/54 | 2/51 |
| 218 | 2/52(0.5mM)<br>2/50(0.3mM) | 20/53(1.0mM)<br>4/50(0.5mM) |
| control | 1/51 | 2/50 |
| 177 | 7/55(0.5mM)<br>1.50(0.25mM) | 12/50(0.1mM)<br>4/50(0.25mM) |
| control | 2/48 | 4/50 |
| 550 | 3/57(0.25mM)<br>2/52(0.1mM) | 8/50(0.1mM)<br>7/50(0.25mM) |
| control | 0/50 | 3/50 |

- to be continued -

Table 2 (continued)

| Compound | Number of cells in which neurites appeared/total number of cells (concentration of the compound) | |
|---|---|---|
| 554 | 6/54(0.25mM)<br>9/50(0.1mM) | 9/50(0.25mM)<br>7/50(0.1mM) |
| control | 1/53 | 3/50 |
| 175 | 10/54(0.5mM)<br>6/50(0.25mM) | 8/53(0.25mM)<br>4/50(0.1mM) |
| control | 1/55 | 3/50 |
| 562 | 8/48(0.5mM)<br>8/56(0.1mM) | 4/51(0.1mM)<br>4/50(0.25mM) |
| control | 3/51 | 2/50 |
| 566 | 19/54(0.5mM)<br>3/50(0.25mM) | 4/53(0.1mM)<br>1/50(0.25mM) |
| control | 2/50 | 0/50 |

- to be continued -

Table 2 (continued)

| Compound | Number of cells in which neurites appeared/total number of cells (concentration of the compound) | |
|---|---|---|
| 218 | 2/52(0.5mM)<br>2/50(0.3mM) | 20/53(1.0mM)<br>4/50(0.5mM) |
| control | 1/51 | 2/50 |
| 177 | 7/55(0.5mM)<br>1/50(0.25mM) | 12/50(0.1mM)<br>4/50(0.25mM) |
| control | 2/48 | 4/50 |
| 550 | 3/57(0.25mM)<br>2/52(0.1mM) | 8/50(0.1mM)<br>7/50(0.25mM) |
| control | 0/50 | 3/50 |
| 554 | 6/54(0.25mM)<br>0/50(0.1mM) | 9/50(0.25mM)<br>7/50(0.1mM) |
| control | 1/53 | 3/50 |
| 175 | 10/54(0.5mM)<br>6/50(0.25mM) | 8/53(0.25mM)<br>4/50(0.1mM) |
| control | 1/55 | 3/50 |
| 546 | 53/58(0.1mM)<br>10/52(0.05mM) | 13/48(0.1mM)<br>3/50(0.05mM) |
| control | 0/50 | 0/50 |
| 557 | 5/52(0.05mM) | 4/50(0.05mM) |
| control | 0/50 | 1/50 |

- to be continued -

**Table 2 (continued)**

| Compound | Number of cells in which neurites appeared/total number of cells (concentration of the compound) | |
|---|---|---|
| 502 | 6/50(1.0mM)<br>2/54(0.5mM) | 12/50(1.0mM)<br>8/50(0.3mM) |
| control | 1/50 | 1/50 |
| 562 | 8/48(0.5mM)<br>8/56(0.1mM) | 4/51(0.1mM)<br>4/50(0.25mM) |
| control | 3/51 | 2/50 |

EXPERIMENTAL EXAMPLE 2

Curative effect on rats with crushed sciatic nerves:-

The curing effect of the compound (I) of the invention was tested on rats having crushed sciatic nerves as a model of peripheral nervous disorder using (1) a change in the action of the hind paw with the crushed sciatic nerves and (2) a change in the weight of the muscle as an index of the course of degeneration and regeneartion of peripheral nerves.

In the experiment, male Wistar rats (6 weeks old), seven per group, were used. The sciatic nerves were crushed by a method similar to the method of Yamatsu et al. (see Kiyomi Yamatsu, Takenori Kaneko, Akifumi Kitahara and Isao Ohkawa, Journal of Japanese Pharmacological Society, 72, 259-268 (1976) and the method of Hasegawa et al. (see Kazuo Hasegawa, Naoji Mikuni and Yutaka Sakai, Journal of Japanese Pharmacological Society, 74, 721-734 (1978). Specifically, under anesthesia with pentobarbital (40 mg/kg, i.p.), the left side sciatic nerve was exposed at the femur and that site of the exposed sciatic nerve which was 5 mm to the center from the branched part between the N. tibialis and the N. suralis was crushed using a modified artery, klomme, having a width of 2 mm and a gap of 0.1 mm. After the operation, the rats were assigned to the test groups at random.

Compound No. 118 was selected as the active ingredient and intraperitoneally administered to the rats once a day from the day of operation to the 22nd day. A group to which mecobalamin (made by Gedeon Richter Ltd.) was administered and a group to which 0.9 % saline was administered were used as controls. The following items were measured with the lapse of time (on the 1st, 4th, 7th, 10th, 14th, 17th, 21st, and 23rd days after the crushing of the sciatic nerves).

(1) Change in the action on the side of the hind paw with the crushed sciatic nerve:-

The distance between digits was measured because this is a good index which functionally shows the degeneration and regeneration of the nerve and its change can be measured with the lapse of time.

By a method similar to the method of Hasegawa [Hasegawa, K., Experientia, 34, 750-751 (1978)], the distance between the first and fifth digits of the hind paw was measured.

The ratio of the measured distance to the normal distance was calculated and expressed in percentage (%). The average calculated values and the standard errors (S. E.) are shown in Table 3. To the values of the test groups which are significantly different, by the t-test of Student, from that of the control group to which physiological saline was administered, superscript * is attached where $p<0.05$ and superscript **, where $p<0.01$.

The distance between the digits was about half (50 %) of the normal distance immediately after the crushing of the sciatic nerve, and tended to decline until the tenth day. No significant difference was seen

among the groups. Regeneration proceeded in the drug-administered groups on the 14th and 17th days, but they showed no significant difference from the group to which saline was administered. On the 21st day, there was an apparent tendency to quicker recovery in the drug-administered groups and the mecobbalamin-administered group, and these groups also show significant differences from the group to which saline was administered. Recovery continued also on the 23rd day.

(2) Change in weight of muscle

It is known that removal of a nerve or its disorder causes atrophy of the muscle which is under its control, and the atrophy is gradually cured by re-control by the nerve. For this reason, a change in the weight of the muscle, which is quantitative, was selected as an index. Twenty-three days after the operation, the soleus muscles of both sides of paws were extracted under anesthesia with pentobarbital, and their weights were measured. The ratio of the weight of the soleus muscle on the crushed side to that of normal side was calculated and expressed in percentage (%). The average values and the standard errors (S. E.) of the groups are shown in Table 3.

**Table 3**

Curative effect with rats crushed in the sciatic nerve

| Drug | Dose (mg/kg, i.p.) | Rate of recovery of the interdigit distance (%) | | Rate of recovery in muscle weight (%) |
|---|---|---|---|---|
| | | 21st day | 23rd day | 23rd day |
| Saline | 1 ml/kg | 62.0±2.4 | 71.1±3.4 | 51.8±1.2 |
| Compound 118 | 30 | 79.8±2.5*** | 87.9±3.3** | 59.6±2.8* |
| Mecobalamin | 0.5 | 79.1±2.6*** | 88.3±4.0** | 55.0±3.5 |

Comparison with the saline-administered group by the Student t-test
*:P<0.05, **<P:0.01, ***:P<0.001

Rats used: Seven per group

EXPERIMENTAL EXAMPLE 3

Promoting effect on the improvement of motor imbalance due to injury of the rat's brain cells by transplantation of fetal cerebral cells:-

Nigral dopaminergic nerve cells at the left side of the brain of 4-week old female Wistar rats (body weight 100 g) were lesioned by injecting a very small quantity of 6-hydroxydopamine. The rats showed a tendency to rotate spontaneously in a direction opposite to the lesioned side for several days, but no

apparent abnormal action was observed after that. Upon administration of methamphethamine (5 mg/kg, i.p.) to the rats having the lesioned nigral dopaminergic nerve cells, they began rotational movement toward the lesioned side.

After two weeks from the destruction by the administration of the drug, portions of the truncus corporis callosi containing dopamine cells (i. e., substantia nigra and the tagmentum at the abdomen side) were cut from the brain of a fetal rat of 14 to 17 days of age, cut finely, and treated with trypsin. Then, the extracted tissues were incubated at 37 °C for 30 minutes, and the tissues were subjected to pipetting to form a suspension. Five microliters of the suspension was transplanted each into two sites of the caudate nucleus of the lesioned side (10 microliters in total, about $10^5$ cells).

Each active ingredient in a dose of 100 mg/kg (i.p.) was administered every day over two weeks from the day of transplantation. The rotational movements induced by administration of methamphetamine were examined 2 weeks and 1 week before, and 2 weeks and 4 weeks after, the transplantation and the administration of the drug. The number of rotational movements within one minute was counted at intervals of 10 minutes after the administration of methamphetamine, and the total number of rotational movements counted six times was averaged to find a mean number of the rotational movements.

The results are shown in Table 4.

Table 4

Effect of the drug on the methamphetamine-induced rotational movement of rats

| | Compound | Number of rotational movements of rats and average values thereof (mean ±S.D.) | | | | |
|---|---|---|---|---|---|---|
| | | Number of weeks after transplantation of nigral dopamine cells | | | | |
| | | -2W | -1W | 2W | 4W | 6W |
| Run No. 1 | 104 | 12.3±3.7 | 10.8±3.8 | 2.0±3.2 | 0.1±0.6 | 0±0 |
| | Saline | 11.0±4.1 | 12.0±6.0 | 3.7±4.7 | 0.4±1.1 | 0.4±2.0 |
| Run No. 2 | 104 | 10.1±6.1 | 10.4±5.2 | 3.1±3.8 | 0.25±1.1 | 1.75±3.0 |
| | 118 | 9.1±5.6 | 10.8±4.9 | 1.9±3.2 | 0.7±1.5 | 0.4±1.1 |
| | Saline | 11.2±4.1 | 11.2±6.2 | 3.7±5.6 | 1.5±3.3 | 2.7±6.4 |
| | | -2W | -1W | 3W | 4W | 6W |
| Run No. 3 | 118 | — | 13.9±7.4 | *5.5±7.2 | -0.1±1.8 | -0.6±2.8 |
| | 554 | — | 14.6±7.6 | *7.0±6.0 | 3.1±3.0 | 0.3±2.3 |
| | Saline | — | 16.7±9.1 | *11.2±9.6 | 5.3±8.3 | 2.8±5.4 |

Rats used: Five to six per group

* Data at 2 W.

EXPERIMENTAL EXAMPLE 4

Improvement of learning and memory of mice with nerve disorder induced by mercury poisoning, and recovery effect:-

Male BalbC strain mice, 7 weeks old, were first caused to learn a T-shaped maze three times in a week so that they run straight from a starting point to a safety area. Then, methylmercury chloride (MMC for short) was administered orally in a dose of 6 mg/kg/day for 6 days. A group of mice to which saline was administered in a dose of 0.1 ml/10 g/day was used as a control group. Beginning with the day next to the day of administering MMC, compounds Nos. 102 and 116 were intraperitoneally administered over 10 days in a dose of 69.5 mg/kg/day and 76.9 mg/kg/day, respectively, so as to make the mole numbers of the compounds equal. On the sixth day after administration of the drug (namely, on the 12th day after start of the experiment), learning of the T-shaped maze was resumed, and the running behaviors of the mice were observed. The number of mice which could be experimented in the T-shaped maze on the 10th and 11th days after the resumption (21st and 22nd days after the start of the experiment) was counted and expressed as a denominator. The number of mice which ran to the safety area within 5 seconds at least 8 times out of ten trial runnings was counted and expressed as a numerator. The decrease in the number of the test animals was due to death by MMC poisoning. The time (seconds) required for the animals to run to the safety area was measured, and the mean ± standard error (SE) was calculated. The results are shown in Table 5.

The results demonstrate the effect of the active ingredient in improving and recovering learning and memory in the mouse.

Table 5

| Improvement of the learning and memory of mice with induced nerve disorder and the recovery effect | | | | |
|---|---|---|---|---|
| Treatment | Number of mice which ran to the safety area within 5 seconds and the running time (seconds) | | | |
| | 10th day | | 11th day | |
| Saline 0.1 ml/10 g/day | 5/6 | 3.0±0.6 | 5/6 | 2.3±0.3 |
| MMC | 4/7 | 2.5±0.4 | 5/7 | 2.1±0.4 |
| MMC + 102 69.5 mg/kg.ip/day | 6/6 | 2.1±0.2 | 6/6 | 3.0±0.6 |
| MMC + 116 76.9 mg/kg.ip/day | 7/7 | 2.1±0.3 | 7/7 | 2.0±0.3 |

EXPERIMENTAL EXAMPLE 5

The acute toxicity of the compounds of the invention was examined by the following method.

Male ddy-strain 5-week old mice and male Wistar-strain 8 week old rats, five per group, were used as experimental animals. Each of the compounds was dissolved in saline and administered perorally (p.o.) or intraperitoneally (i.p.), and the toxicity of the compound was assessed 24 hours after the administration. The results are shown in Tables 6 and 7.

Table 6

| Acute toxicity ($LD_{50}$) in mouse | | | |
|---|---|---|---|
| Compound | Number of dead animals/number of animals tested | | Estimated $LD_{50}$ (mg/kg, p.o.) |
| | Dose (mg/kg, p.o.) | | |
| | 550 | 1000 | |
| 402 | 0/5 | 3/5 | >1000 |
| 128 | 0/5 | 0/5 | >1000 |
| 112 | - | 0/5 | >1000 |
| 102 | - | 0/5 | >1000 |
| 118 | - | 0/5 | >1000 |
| 108 | - | 2/5 | >1000 |
| 122 | - | 1/5 | >1000 |
| 406 | - | 0/5 | >1000 |
| 216 | 0/5 | - | >550 |
| 218 | 0/5 | - | >550 |
| 126 | - | 0/5 | >1000 |
| - : Not tested | | | |

Table 7

| Acute toxicity ($LD_{50}$) in mouse | |
|---|---|
| Compound | Estimated $LD_{50}$ (mg/kg, i.p.) |
| 118 | 500-1000 |
| 117 | 250-500 |
| 554 | 250-500 |
| 175 | 500-1000 |
| 570 | <125 |
| 550 | <500 |
| 562 | 500-1000 |
| 546 | 500-1000 |
| 557 | 500-1000 |
| 559 | 500-1000 |
| 566 | 500-1000 |
| 574 | 500-1000 |
| 578 | 500-1000 |
| 582 | 500-1000 |
| 502 | 500-1000 |

## Claims

**1.** A compound which is a pyrimidine derivative of formula (I)

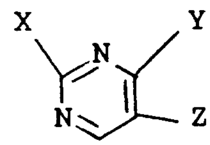

...(I)

wherein X is either

(i) a group of formula (I)-1

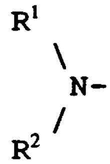

...(I)-1

wherein $R^1$ represents a hydrogen atom or an alkyl group having 1 to 4 carbon atoms, $R^2$ represents a piperidyl group which may be substituted by a $C_{1-4}$ alkyl group, a phenethyl, cyclohexyl, phenyl or benzyl group or an alkyl group having 1 to 4 carbon atoms which may be substituted by a piperidino group, or $R^1$ and $R^2$ together with the nitrogen atom to which they are attached may form a heterocyclic ring selected from

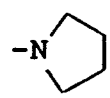 , 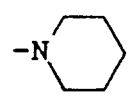 , 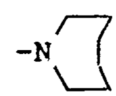 ,

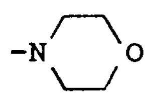 , 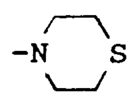 , 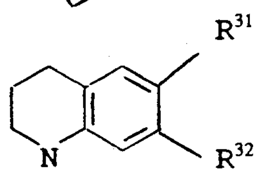 ,

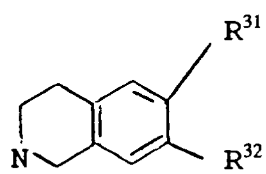 and 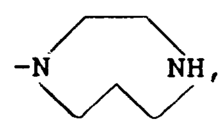 ,

wherein $R^{31}$ and $R^{32}$, which may be the same or different, each represents a hydrogen atom or an alkoxy group having 1 to 4 carbon atoms, and the heterocyclic group may optionally be substituted by a phenyl, benzyl, phenylthio, cyano or alkoxycarbonyl group having 1 to 4 carbon atoms or mono-substituted by the group

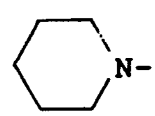

or mono- to penta-substituted by a $C_{1-4}$ alkyl group, or substituted by a $C_{3-5}$ polymethylene group on the adjoining ring-member carbons, or

(ii) a group of formula (I)-2

$-S-R^4$ (I)-2

wherein $R^4$ represents an alkyl group having 1 to 4 carbon atoms,
Y represents an amino group or a substituted amino group monoor di-substituted by a $C_{1-4}$ alkyl group, and Z represents a methyl group substituted by a $C_{2-5}$ alkoxycarbonyl group or an alkoxycarbonyl group having 2 to 5 carbon atoms, or Y and Z together may form a divalent group -Y-Z- of formula

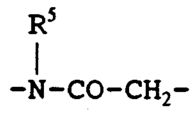

wherein $R^5$ represents an alkyl group having 1 to 4 carbon atoms which may be substituted by an alkoxy group having 1 to 4 carbon atoms, or a group

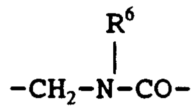

wherein $R^6$ represents an alkyl group having 1 to 4 carbon atoms provided that when Y is an amino group or a substituted amino group mono- or di-substituted by a $C_{1-4}$ alkyl group, X is

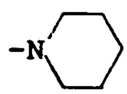 or 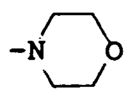;

or a pharmaceutically acceptable salt thereof.

2. A compound according to claim 1 wherein the salt is a hydrochloride, hydrobromide, sulfate, bisulfite, phosphate, acid phosphate, acetate, maleate, fumarate, succinate, lactate, tartrate, benzoate, citrate, gluconate, glucanate, methanesulfonate, p-toluenesulfonate, naphthalenesulfonate or quaternary ammonium salt.

3. A therapeutic agent for use in the treatment of neurological diseases which comprises as an active ingredient a compound as claimed in claim 1 or 2.

4. A pharmaceutical composition which comprises as active ingredient, a compound as claimed in claim 1 or 2 in association with a pharmaceutically acceptable diluent.

5. A process for producing a compound as claimed in claim 1, which comprises
(a) reacting a compound of formula (V)

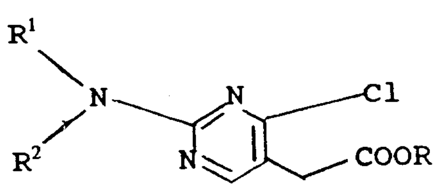

. . . (V)

wherein $R^1$ and $R^2$ are as defined in claim 1, and R is an alkyl group having 1 to 4 carbon atoms, with an amine of formula (VI)

$R^5NH_2$ (VI)

wherein $R^5$ is as defined in claim 1 to produce a compound of formula (I)-A

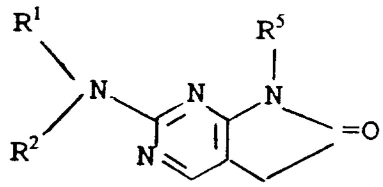

...(I)-A;

or

($b^1$) reacting a compound of formula (XII)

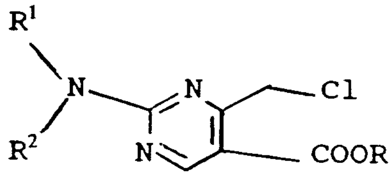

...(XII)

wherein $R^1$ and $R^2$ are as defined in claim 1, and R represents an alkyl group having 1 to 4 carbon atoms,

with a compound of formula (XIII)

$R^6NH_2$ (XIII)

wherein $R^6$ is as defined in claim 1 or

($b^2$) reacting a compound of formula (XIV)

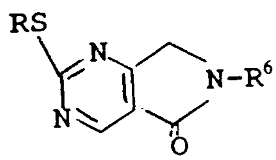

...(XIV)

wherein $R^6$ is defined in claim 1 and R is an alkyl group having 1 to 4 carbon atoms,

with a compound of formula (XV)

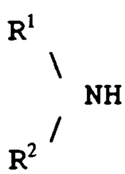

...(XV)

wherein $R^1$ and $R^2$ are as defined in claim 1 to produce a compound of formula (I)-B

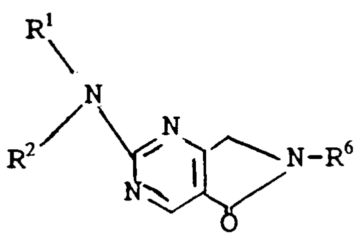

...(I)-B

wherein $R^1$, $R^2$ and $R^6$ are as defined in claim 1; or

(c) reacting a compound of formula (XVI)

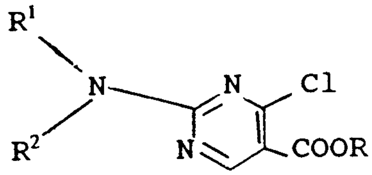

...(XVI)

wherein $R^1$ and $R^2$ together with the nitrogen atom to which they are attached form a group

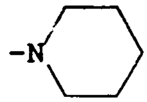

or a group

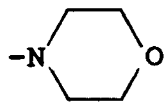

and R is as defined above, with ammonia to produce a compound of formula (I)-C

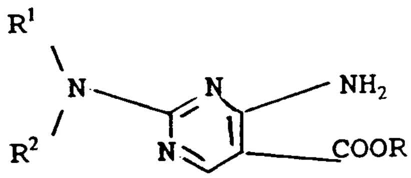

...(I)C;

or

(d) reacting a compound of formula (V) wherein $R^1$ and $R^2$ together with the nitrogen atom to which they are attached form a group

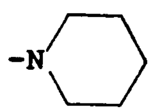

or a group

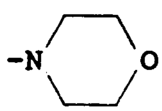

with a compound of formula (XVII)

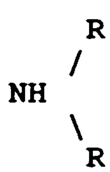

...(XVII)

wherein the R's independently represent an alkyl group having 1 to 4 carbon atoms,

to produce a compound of formula (I)-D

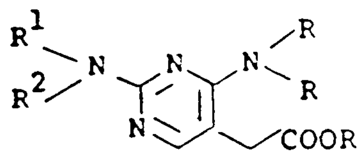

...(I)-D

and, if desired, converting the compound of formula (I)-A, (I)-B, (I)-C or (I)-D into a pharmaceutically acceptable salt.

**Patentansprüche**

1. Verbindung, welche ein Pyrimidinderivat der Formel (I)

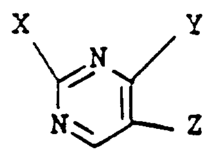

...(I)

ist, worin X entweder
(i) eine Gruppe der Formel (I) - 1

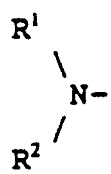

...(I)-1

ist, worin $R^1$ für ein Wasserstoffatom oder eine Alkylgruppe mit 1 bis 4 Kohlenstoffatomen steht, $R^2$ eine Pyperidylgruppe, die mit einer $C_{1-4}$-Alkylgruppe, einer Phenethylgruppe, einer Cyclohexylgruppe, einer Phenylgruppe oder Benzylgruppe substituiert sein kann, oder eine Alkylgruppe mit 1 bis 4 Kohlenstoffatomen, die mit einer Piperidinogruppe substituiert sein kann, bedeutet, oder worin $R^1$ und $R^2$ zusammen mit einem Stickstoffatom, an welches sie gebunden sind, einen heterocyclischen Ring, ausgewählt aus

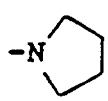 , 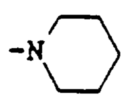 , 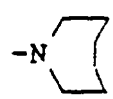 ,

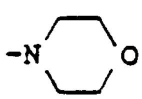 , 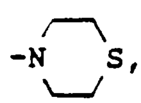 , 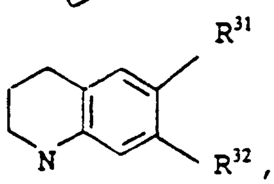 ,

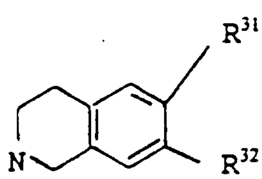 und 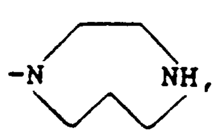 ,

bilden können, worin $R^{31}$ und $R^{32}$, welche gleich oder unterschiedlich sein können, jeweils ein Wasserstoffatom oder eine Alkoxygruppe mit 1 bis 4 Kohlenstoffatomen bedeuten, und die heterocyclische Gruppe wahlweise mit einer Phenyl-, Benzyl-, Phenylthio, Cyano- oder Alkoxycarbonylgrupppe mit 1 bis 4 Kohlenstoffatomen substituiert sein kann, oder mit der Gruppe

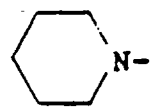

mono-substituiert, oder mit einer $C_{1-4}$-Alkylgruppe mono- bis penta-substituiert sein kann, oder mit einer $C_{3-5}$-Polymethylengrupe an den angrenzenden Ringkohlenstoffatomen substituiert sein kann; oder
(ii) eine Gruppe der Formel (I) - 2

$-S-R^4$ (I) - 2

worin $R^4$ für eine Alkylgruppe mit 1 bis 4 Kohlenstoffatomen steht,
Y eine Aminogruppe oder eine substituierte Aminogruppe, mono- oder di-substituiert mit einer $C_{1-4}$-Alkylgruppe, bedeutet, und Z für eine mit einer $C_{2-5}$-Alkoxycarbonylgruppe substituierte Methylgruppe oder eine Alkoxycarbonylgruppe mit 2 bis 5 Kohlenstoffatomen steht, oder worin Y und Z zusammen eine bivalente Gruppe -Y-Z- der Formel

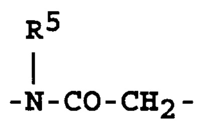

bilden, worin $R^5$ für eine Alkylgruppe mit 1 bis 4 Kohlenstoffatomen, welche mit einer Alkoxygruppe mit 1 bis 4 Kohlenstoffatomen substituiert sein kann, oder für eine Gruppe der Formel

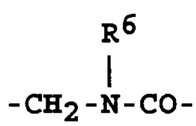

steht, worin $R^6$ eine Alkylgruppe mit 1 bis 4 Kohlenstoffatomen bedeutet, mit der Maßgabe, daß X

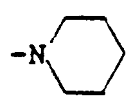 oder 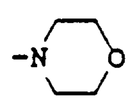

ist, wenn Y eine Aminogruppe oder eine substituierte Aminogruppe, mit einer $C_{1-4}$-Alkylgruppe mono- oder di-substituiert, ist; oder ein pharmazeutisch annehmbares Salz davon ist.

2. Verbindung nach Anspruch 1, worin das Salz ein Hydrochlorid-, Hydrobromid-, Sulfat-, Bisulfit-, Phosphat-, saures Phosphat-, Acetat-, Maleat-, Fumarat, Succinat-, Lactat-, Tartrat-, Benzoat-, Citrat-, Gluconat-, Glucanat-, Methansulfonat-, p-Toluolsulfonat-, Naphthalinsulfonat-, oder ein quaternäres Ammoniumsalz ist.

3. Therapeutisches Mittel zur Verwendung bei der Behandlung von neurologischen Erkrankungen, welches als aktiven Bestandteil eine Verbindung nach Anspruch 1 oder 2 umfaßt.

4. Pharmazeutische Zusammensetzung, welche als aktiven Bestandteil eine Verbindung nach Anspruch 1 oder 2 in Verbindung mit einem pharmazeutisch annehmbaren Verdünnungsmitttel umfaßt.

5. Verfahren zur Herstellung einer Verbindung nach Anspruch 1, welches

(a) die Umsetzung einer Verbindung der Formel (V)

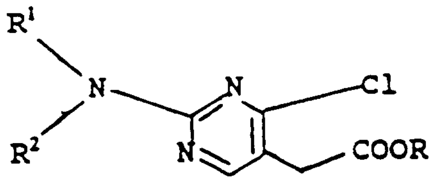

... (V)

worin $R^1$ und $R^2$ wie in Anspruch 1 definiert sind, und R eine Alkylgruppe mit 1 bis 4 Kohlenstoffatomen ist,
mit einem Amin der Formel (VI)

$R^5NH_2$ (VI)

worin $R^5$ wie in Anspruch 1 definiert ist, zur Herstellung einer Verbindung der Formel (I)-A

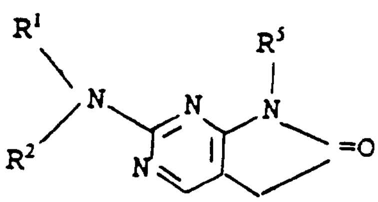

...(I) - A;

oder
($b^1$) die Reaktion einer Verbindung der Formel (XII)

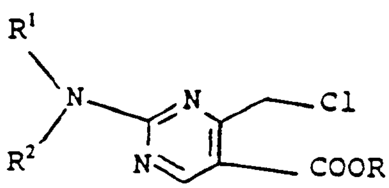

... (XII)

worin $R^1$ und $R^2$ wie in Anspruch 1 definiert sind, und R für eine Alkylgruppe mit 1 bis 4 Kohlenstoffatomen steht,
mit einer Verbindung der Formel (XIII)

$R^6NH_2$ (XIII)

worin $R^6$ wie in Anspruch 1 definiert ist; oder
($b^2$) die Reaktion einer Verbindung der Formel (XIV)

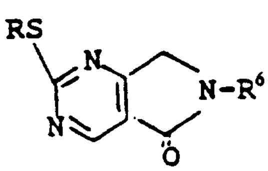

... (XIV)

worin $R^6$ wie in Anspruch 1 definiert ist, und R eine Alkylgruppe mit 1 bis 4 Kohlenstoffatomen ist,
mit einer Verbindung der Formel (XV)

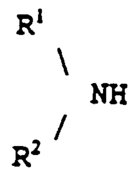

... (XV)

worin $R^1$ und $R^2$ wie in Anspruch 1 definiert sind, zur Herstellung der Verbindung der Formel (I) - B

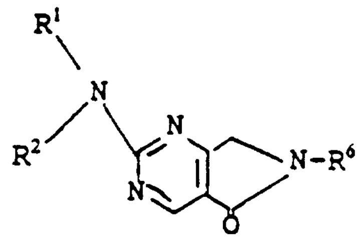

... (I) -B

worin $R^1$, $R^2$ und $R^6$ wie in Anspruch 1 definiert sind; oder
(c) die Reaktion einer Verbindung der Formel (XVI)

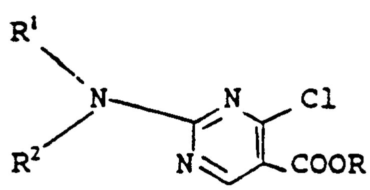

... (XVI)

worin $R^1$ und $R^2$ zusammen mit einem Stickstoffatom, mit dem sie verbunden sind, eine Gruppe

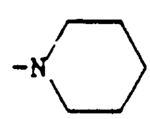

oder eine Gruppe

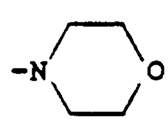

bilden, und R wie oben definiert ist, mit Ammonik zur Herstellung einer Verbindung der Formel (I) - C

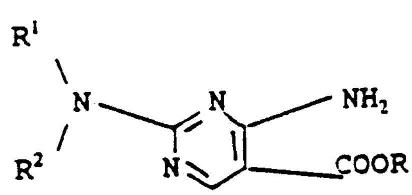

... (I)-C;

oder
(d) die Reaktion einer Verbindung der Formel (V), worin $R^1$ und $R^2$ zusammen mit einem Stickstoffatom, mit dem sie verbunden sind, eine Gruppe

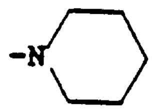

oder eine Gruppe

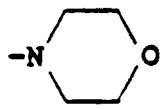

bilden, mit einer Verbindung der Formel (XVII)

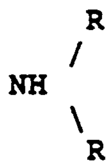

...(XVII)

worin die R's unabhängig voneinander für eine Alkylgruppe mit 1 bis 4 Kohlenstoffatomen stehen, zur Herstellung einer Verbindung der Formel (I) - D

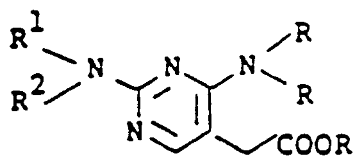

...(I)-D

und, falls erwünscht, die Umwandlung der Verbindung der Formel (I) - A, (I)-B, (I) - C oder (I) - D in ein pharmazeutisch annehmbares Salz umfaßt.

**Revendications**

**1.** Composé qui est un dérivé de pyrimidine de formule (I) :

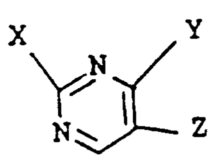

(I)

dans laquelle X représente :
(i) un groupe de formule (I)-1 :

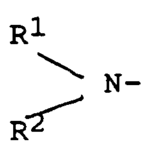

(I)-1

dans laquelle $R^1$ représente un atome d'hydrogène ou un groupe alkyle ayant 1 à 4 atomes de

carbone, $R^2$ représente un groupe pipéridyle qui peut être substitué par un groupe alkyle en $C_1$ à $C_4$, un groupe phénéthyle, cyclohexyle, phényle ou benzyle, ou un groupe alkyle ayant 1 à 4 atomes de carbone, qui peut être substitué par un groupe pipéridino, ou bien $R^1$ et $R^2$ peuvent former avec l'atome d'azote auquel ils sont reliés, un noyau hétérocyclique choisi parmi :

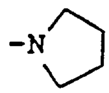 , 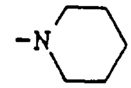 , 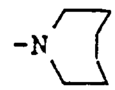 ,

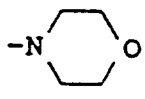 , 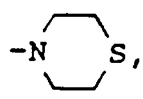 , 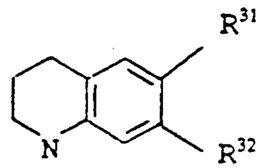 ,

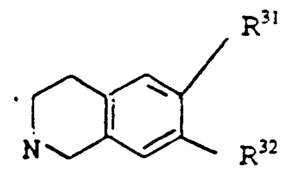 et 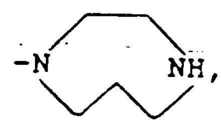 ,

$R^{31}$ et $R^{32}$, qui peuvent être identiques ou différents, représentant chacun un atome d'hydrogène ou un groupe alcoxy ayant 1 à 4 atomes de carbone, et le groupe hétérocyclique pouvant être éventuellement substitué par un groupe phényle, benzyle, phénylthio, cyano ou alcoxycarbonyle ayant 1 à 4 atomes de carbone, ou monosubstitué par le groupe

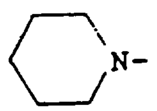 ,

ou mono- à pentasubstitué par un groupe alkyle en $C_1$ à $C_4$, ou substitué par un groupe polyméthylène en $C_3$ à $C_5$ sur les atomes de carbone voisins du noyau,
ou (ii) un groupe de formule (I)-2 :

$-S-R^4$ (I)-2,

dans laquelle $R^4$ représente un groupe alkyle ayant 1 à 4 atomes de carbone,
Y représente un groupe amino ou un groupe amino substitué, monosubstitué ou disubstitué par un groupe alkyle en $C_1$ à $C_4$, et Z représente un groupe méthyle substitué par un groupe alcoxycarbonyle en $C_2$ à $C_5$, ou un groupe alcoxycarbonyle ayant 2 à 5 atomes de carbone, ou bien Y et Z peuvent former ensemble un groupe divalent -Y-Z- de formule :

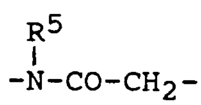

dans laquelle $R^5$ représente un groupe alkyle ayant 1 à 4 atomes de carbone, qui peut être substitué par un groupe alcoxy ayant 1 à 4 atomes de carbone, ou un groupe divalent de formule :

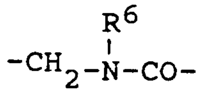

dans laquelle $R^6$ représente un groupe alkyle ayant 1 à 4 atomes de carbone,
étant entendu que, lorsque Y représente un groupe amino ou un groupe aminosubstitué, mono- ou disubstitué par un groupe alkyle en $C_1$ à $C_4$, X représente

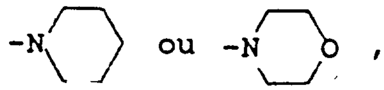

ou un sel pharmaceutiquement acceptable d'un tel dérivé.

2. Composé selon la revendication 1 sous forme de sel, qui est un chlorhydrate, un bromhydrate, un sulfate, un bisulfite, un phosphate, un phosphate acide, un acétate, un maléate, un fumarate, un succinate, un lactate, un tartrate, un benzoate, un citrate, un gluconate, un glucanate, un méthanesulfonate, un p-toluènesulfonate, un naphtalènesulfonate ou un sel d'ammonium quaternaire.

3. Agent thérapeutique destiné à être utilisé dans le traitement d'affections neurologiques, qui comprend, en tant qu'ingrédient actif, un composé tel que revendiqué dans la revendication 1 ou 2.

4. Composition pharmaceutique qui comprend un composé tel que revendiqué dans la revendication 1 ou 2, en tant qu'ingrédient actif, ledit composé étant associé à un diluant pharmaceutiquement acceptable.

5. Procédé de préparation d'un composé tel que revendiqué dans la revendication 1, qui comprend :
(a) la réaction d'un composé de formule (V) :

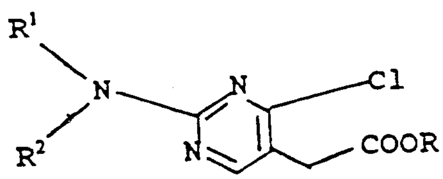

(V)

dans laquelle $R^1$ et $R^2$ sont tels que définis dans la revendication 1 et R représente un groupe alkyle ayant 1 à 4 atomes de carbone,
avec une amine de formule (VI) :

$R^5NH_2$ (VI)

dans laquelle $R^5$ est tel que défini dans la revendication 1, pour la production d'un composé de formule (I)-A :

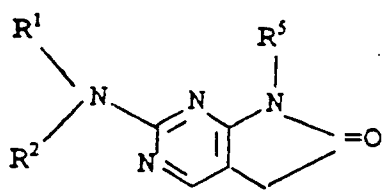

(I)-A,

ou
($b^1$) la réaction d'un composé de formule (XII) :

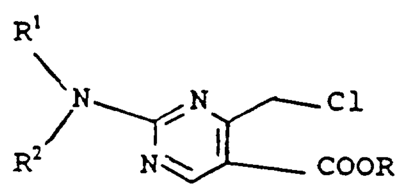

(XII)

dans laquelle $R^1$ et $R^2$ sont tels que définis dans la revendication 1 et R représente un groupe alkyle

ayant 1 à 4 atomes de carbone,
avec un composé de formule (XIII) :

$R^6NH_2$ (XIII)

dans laquelle $R^6$ est tel que défini dans la revendication 1,
ou
($b^2$) la réaction d'un composé de formule (XIV) :

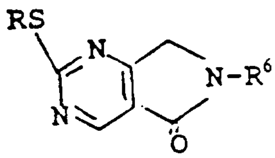

(XIV)

dans laquelle $R^6$ est tel que défini dans la revendication 1 et R représente un groupe alkyle ayant 1 à 4 atomes de carbone,
avec un composé de formule (XV) :

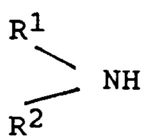

(XV)

dans laquelle $R^1$ et $R^2$ sont tels que définis dans la revendication 1, pour la production d'un composé de formule (I)-B :

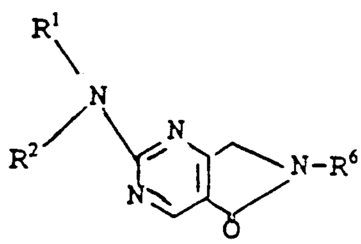

(I)-B

dans laquelle $R^1$, $R^2$ et $R^6$ sont tels que définis dans la revendication 1,
ou
(c) la réaction d'un composé de formule (XVI) :

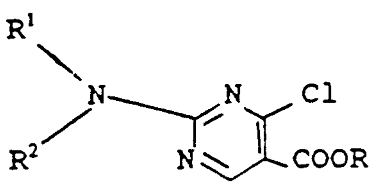

(XVI)

dans laquelle $R^1$ et $R^2$ forment, avec l'atome d'azote auquel ils sont liés, un groupe

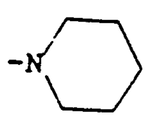

ou un groupe

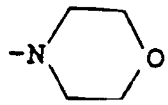

,

et R est tel que défini précédemment, avec de l'ammoniac pour la production d'un composé de formule (I)-C :

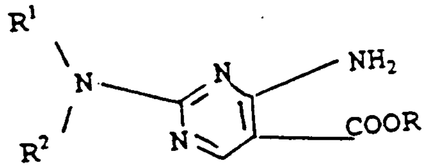

(I)-C,

ou

(d) la réaction d'un composé de formule (V) dans laquelle $R^1$ et $R^2$ forment, avec l'atome d'azote auquel ils sont liés, un groupe

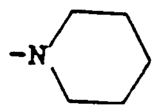

ou un groupe

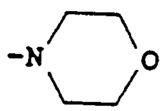

,

avec un composé de formule (XVII) :

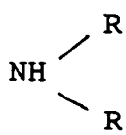

(XVII)

dans laquelle les groupes R représentent chacun, indépendamment, un groupe alkyle ayant 1 à 4 atomes de carbone, pour la production d'un composé de formule (I)-D :

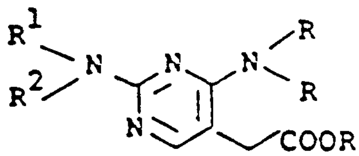

(I)-D,

et, si on le souhaite, la transformation du composé de formule (I)-A, (I)-B, (I)-C ou (I)-D en un sel pharmaceutiquement acceptable.